# EUROPEAN PATENT APPLICATION

(11) **EP 4 847 005 A1**
(43) Date of publication of application: **02.12.2026**
(21) Application number: 25179244.6
(22) Date of filing: 27.05.2025
(51) Int. Cl.: A61K 8/34, A61K 8/365, A61K 8/41, A61K 8/86, A61Q 5/06, A61K 8/73

(54) **METHOD FOR THE SEMI-PERMANENT DEFORMATION OF KERATIN FIBERS**

(71) Applicant: Wella Germany GmbH, 64295 Darmstadt (DE)
(72) Inventor: Alam, Ehsanul, Darmstadt (DE); Schmitt, Manfred, Darmstadt (DE); Kaestle, Katrin, 64295 Darmdstadt (DE); Schmidt, Carsten, 64295 Darmstadt (DE); Telser, Sonja, 64295 Darmstadt (DE)
(74) Representative: Zimmermann & Partner Patentanwälte mbB

(57) **Abstract**

The invention relates to a method for the semi-permanent deformation of hair, with reduced formaldehyde exposure. The invention further relates to a composition for use in the method.

## Description

### FIELD OF THE INVENTION

The present invention relates to methods for the semipermanent deformation of hair, cosmetic compositions for use in the method, and the use of the method and of the compositions, respectively, for reducing formaldehyde exposure in the semipermanent deformation of hair. The cosmetic composition comprises at least one keto-compound (a), or derivative thereof, at least one surfactant (b), at least one thickener (c), at least one fatty alcohol (d), and at least one surfactant (e) different from (b) and (d), and an aqueous carrier.

### BACKGROUND OF THE INVENTION

In replacement of conventional methods for the permanent deformation of hair shaping, method for the semi-permanent deformation of hair comprising alpha-ketoacids are known. See for example, the PCT application WO2014/072491A1 published on 15 May 2014, the European applications EP3973944A1 and EP3973945A1 published on 30 March 2022, the PCT application WO2025/019781A1 published on 23 January 2025.

Usually, in order to achieve satisfactory benefits in terms of straightening and antifrizziness, complex formulations and chemical processes are used. Even though a satisfactory straightening and anti-frizziness may be achieved with known compositions, these compositions may impair hair shine and manageability. These compositions may also impart a stinging and itching sensation, after carrying out the flat-iron treatment. These compositions may also leave unwanted residues on hair. These compositions may also have a significant damage profile. In order to compensate these drawbacks and therefore to achieve consumer acceptance, additional compounds are usually incorporated to the compositions, which complexify them further. Various aspects including the incorporation of alpha-ketoacids at a significant amount, the acidic pH of the composition, the incorporation of additional compounds, etc. are associated with additional drawbacks, as they impair the sustainability of the composition and they also alter the hairdresser and/or consumer's experience during the flat-iron treatment, because of the generation of a significant amount of fume.

There are several concerns, however, regarding keratin treatment compositions containing alpha-ketoacids such as glyoxylic acid (GA) as active ingredient are. Particular concerns relate to the known release of formaldehyde by such compositions, during the high-temperature ironing process (up to 230°C) that is essential for best product performance. There is therefore the need for providing a method for the semipermanent deformation of hair, which method allows for controlling the exposure of users and stylists to formaldehyde during the semipermanent deformation of hair. According to preferred embodiments, the method will also be suitable for reducing the formaldehyde exposure of users and stylists during the semipermanent deformation of hair.

There is also a need for providing a cosmetic composition suitable for use in the method for the semi-permanent deformation of keratin fibers, particularly human hair. There is also the need for providing a composition, which may be used in the so-called *"Brazilian hair straightening method".* There is also the need for providing a composition, which has a broad range of activity, from eliminating hair frizz to straightening, reducing volume and better definition of curls and waves. There is also the need for providing a composition, which is suitable for the semi-permanent deformation of hair, while providing superior shine to hair. There is also the need for providing a composition, which is suitable for the semi-permanent deformation of hair, while providing superior manageability to hair. There is also the need for providing a composition, which is suitable for the semi-permanent deformation of hair, while providing superior conditioning to hair. There is also the need for providing a composition, which is suitable for the semi-permanent deformation of hair, while imparting a reduced stinging and itching sensation or even preventing any stinging and itching sensation. There is also the need for providing a composition, which is suitable for the semi-permanent deformation of hair, while having a very low damage profile or even a no damage profile. There is also the need for providing a composition, which is suitable for the semi-permanent deformation of hair, while having a satisfactory sustainability, particularly by being an eco-ethical formulation. There is in particular the need for providing a composition, which is suitable for the semi-permanent deformation of hair, while providing for low formaldehyde exposure during the semi-permanent deformation of hair.

Hence, there is a need for providing a composition, which is suitable for the semipermanent deformation of hair, that imparts the benefits of hair compositions to the hair that are conventionally used for semi-permanent deformation such as of hair shine, hair manageability, hair conditioning and damage profile, while generating a minimal amount of fume during the application of the hair composition. In particular, there is need for a composition that generates a minimal amount of formaldehyde during the application of the hair composition, i.e. during the semi-permanent deformation of hair.

Accordingly, it is an object of the presently claimed invention to provide a composition, which is suitable for the semi-permanent deformation of hair, that imparts of hair compositions to the hair that are conventionally used for semi-permanent deformation such as of hair shine, hair manageability, hair conditioning and damage profile, while generating a minimal amount of fume during the application of the hair composition, in particular while generating a minimal amount of formaldehyde during the application of the hair composition.

### SUMMARY OF THE INVENTION

Subject matter of the present invention is a method for the semipermanent deformation of hair as defined in claim 1. The dependent claims relate to particular embodiments thereof. Further subject matter of the present invention is a composition for the semipermanent deformation of hair, which composition is suitable for use in the method according to the invention. Further subject matter of the present invention is the method, and of the composition, respectively, for controlling the fume generation and/or formaldehyde release during the semipermanent deformation of hair.

Surprisingly, it was found that the method for the semi-permanent deformation of hair according to the present invention allows for controlling the generation of fume and/or formaldehyde release (or exposure) within acceptable levels. The method according to the present invention comprises the following steps:
(A) applying a cosmetic composition onto hair,
(B) leaving the composition on the hair for a suitable application time,
(C) rinsing off the composition from the hair, and
(D) applying heat onto the hair using a heating device.

The composition applied onto hair in step (A) comprises:
(a) at least one keto-compound selected from alpha-ketoacids, alpha-ketoacid esters, alpha-ketoacid amides, glyoxyloyl-derivatized compounds, or mixtures thereof,
(b) at least one surfactant selected from mono-tail or double-tail fatty amidoamines or esteramines, quaternary fatty ammonium compounds, quaternary fatty amidoammonium or esterammonium compounds, fatty imidazolines, complex quaternary ammonium compounds, quaternary ammonium compounds of PEG-ylated carbohydrate derivatives,
(c) at least one thickener selected from guar gum, xanthan gum, carrageenan, pectin, agar, locust bean gum, sclerotium gum, tara gum, gellan gum, konjac gum, arabic gum, alginate, tragacanth gum, karaya gum, diutan gum,
(d) at least one fatty alcohol, and
(e) at least one surfactant different from component (b) and (d),
(f) an aqueous carrier.

It is presently understood that the desired control of fume generation and/or formaldehyde release/ exposure is achieved by a combination of method steps and product features resulting from the particular composition applied onto the hair.

In addition to the desired control of fume and formaldehyde release upon use in flat-iron treatments, the methods and compositions according to the present invention allow for maintaining the straightening / smoothening performance provided by the keto-compounds, without compromising the expected benefits, particularly in terms of hair shine, hair manageability, hair conditioning and damage profile. Without wishing to be bound by any theory, the inventors believe that the satisfactory performance of the present invention may be due to the physical and/or chemical interactions of compounds upon application of the composition to hair. On one hand, upon application on hair, components present in the composition may form hydrogen bonds or associative bonds between each other and/or functional groups present on the hair (physical interactions). On the other hand, excess components and components that already have fulfilled their function and are no longer essential are subsequently rinsed-off from the hair. Upon further application of heat, particularly using a heating device, the components present on the hair may further form covalent bonds between each other and/or functional groups present on the hair (chemical interaction).

### DEFINITIONS

As used in the specification and the appended claims, the singular forms "a," "an" and "the" include plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The term "may" in the context of this application means "is permitted to" or "is able to" and is a synonym for the term "can." The term "may" as used herein does not mean possibility or chance.

The term "and/or" in the context of this application means one or the other or both. For example, an aqueous solution of A and/or B means an aqueous solution of A alone, an aqueous solution of B alone and an aqueous solution of a combination of A and B.

The term "about" is understood to mean ±10 percent of the recited number, numbers or range of numbers. According to embodiments, the term "about" is understood to mean ±2 percent of the recited number, numbers or range of numbers.

As used herein, the term "optionally" means that the corresponding step or feature may or may not be present. It includes both possibilities.

As used herein, the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Unless specifically indicated otherwise, all values, ranges, percentages etc. in the present disclosure are "by-weight". Further, for a given entity such as for example a "composition", the total amount of respective components (the amount of which is indicated herein in terms of percent-by-weight ranges) adds up to 100 percent-by-weight.

The term "composition" as used herein denotes a mixture or blend comprising at least two components. Typical compositions comprise at least one functional component (such as, for example, an active ingredient) and at least one solvent component (such as water or an alcohol). A composition may be present in any suitable physical form, including for example liquids of varying viscosities, gels, foams, creams, and the like. Typically, the compositions according to the present invention are liquids (including solutions and dispersions). The term "composition" may denote a ready-to-use product unless the context dictates otherwise.

By "a cosmetic composition for the semi-permanent deformation of keratin fibers" is meant a hair composition, which is suitable for changing the waviness and frizziness of hair, albeit not permanently. Such composition is referred hereinafter as "the composition" or "the composition of the presently claimed invention", unless otherwise specified. The composition of the presently claimed invention may be used for straightening, relaxing and/or smoothing keratin fibers, preferably human hair. In contrast, the composition of the presently claimed invention is not suitable for use as a permanent shaping composition i.e., a cosmetic composition for the permanent deformation (i.e., waving) of keratin fibers, preferably human hair.

Ranges defined throughout the specification include the end values, i.e. a range of from 1 to 10 implies that both 1 and 10 are included in the range. For the avoidance of doubt, the applicant shall be entitled to any equivalents according to applicable law.

The term "essentially free" of a particular component denotes an amount of less than 1.0% by weight of the respective component in an entity such as a composition, based on the total weight of the entity. In particular, the term "essentially free" denotes an amount of less than 0.5% by weight, for example less than 0.1% by weight, such as less than 0.01% by weight of the respective component in the entity.

The term "free" of a particular component denotes an amount below the detection limit, assuming that detectability is on a ppm (parts per million) basis (by weight). In particular, the term "free" of a particular component denotes an amount below 50 ppm-by-weight, for example less than 10 ppm-by-weight, such as less than 5 ppm-by-weight of the respective component in the entity.

The term "consisting essentially of" denotes the absence of additional, not specifically recited components in an entity such as a composition in an amount of more than 1.0% by weight, based on the total weight of the entity. In particular, the term "consisting essentially of" denotes the absence of additional, not specifically recited components in an amount of more than 0.5% by weight, for example more than 0.1% by weight, such as more than 0.01% by weight in an entity. It goes without saying that any additional, not specifically recited component is compatible with the specifically recited components and does not interfere with the function thereof.

The term "consisting of" denotes the absence of additional, not specifically recited components in an entity such as a composition in an amount amount below the detection limit, assuming that detectability is on a ppm (parts per million) basis (by weight). In particular, the term "consisting of" denotes the absence of additional, not specifically recited components in an amount of more than 50 ppm-by-weight, for example more than 10 ppm-by-weight, such as more than 10 ppm-by-weight in an entity. It goes without saying that any additional, not specifically recited component is compatible with the specifically recited components and does not interfere with the function thereof.

The term "hair" as used herein generally may denote "living hair", i.e., on a living body, or "nonliving hair", i.e., in a wig, hairpiece or other aggregation of non-living keratinous fibers. In particular, the term "hair" as used herein denotes mammalian hair, more particularly human hair. Wool, fur and other keratin fibers (keratin-containing fibers) are encompassed, however, by the term "hair" as used herein. More specifically, for the purposes of the present invention the term "hair" denotes human "head hair" or "scalp hair", and "applying to scalp" is intended to encompass applying the compositions of the present invention to (at least part of the) scalp region.

"Ready-to-use scalp-and-hair treatment product " denotes the form in which the composition is actually applied to human scalp, or to a skin region.

Reference throughout this specification to "one embodiment" or "preferred embodiment" means that a particular feature, structure, or characteristic described in connection with the embodiment is included in at least one embodiment of the present invention. Thus, appearances of the phrases "in one embodiment" or "in a preferred embodiment" in various places throughout this specification are not necessarily all referring to the same embodiment but may refer to different embodiments of the present invention. Furthermore, the features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the subject matter, and form different embodiments, as would be understood by those in the art. For example, in the appended claims, any of the claimed embodiments can be used in any combination.

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests, or discloses any such invention. Further, to the extent that any meaning or definition of a term in this document conflicts with any meaning or definition of the same term in a document incorporated by reference, the meaning or definition assigned to that term in this document shall govern.

Measurement techniques described herein are well known to a person skilled in the art and therefore do not limit the present invention.

By "user" or "customer" it is meant the person preparing the hair treatment composition. The user is for example a professional hair stylist working in a salon and is different from the subject on whose hair the composition is applied, or the user is identical to the person on whose hair the composition is applied. The mixing can take place in a bowl or within a bottle or even through a mixer placed between the stored individual compositions and the point at which it is dispensed to be used. While two component bleaching media are the most commonly found in the market, there are also other bleaching media which use three or more components.

In the following disclosure, different aspects of the subject matter are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Materials denoted below are being used in the embodiments of the present invention. The present invention is described with non-limiting embodiments. Listed ingredients typically are preferred features as well as other preferred but non-limiting features of the present invention.

### DETAILED DESCRIPTION OF THE INVENTION

Before the methods and compositions / formulations of the present invention are described, it is to be understood that this invention is not limited to particular methods and compositions / formulations described, since such methods and compositions / formulations may, of course, vary. It is also to be understood that the terminology used herein is not intended to be limiting, since the scope of the presently claimed invention will be limited only by the appended claims.

Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the presently claimed invention, and form different embodiments, as would be understood by those in the art. For example, in the appended claims, any of the claimed embodiments can be used in any combination.

### 1 Methods for the semi-permanent deformation of keratin fibers, or hair

In one aspect, the present invention relates to methods for the semi-permanent deformation of keratin fibers. In particular, the methods according to the present invention are for the semi-permanent deformation of hair, and preferably human hair. The methods according to the present invention comprise the following steps:
(A) applying a cosmetic composition onto hair,
(B) leaving the composition on the hair for a suitable application time,
(C) rinsing-off the composition from the hair, and
(D) applying heat onto the hair using a heating device.

According to step (A) of the methods according to the present invention, a cosmetic composition is applied onto hair. The cosmetic composition is a composition for the semi-permanent deformation of keratin fibers. The cosmetic composition comprises:
(a) at least one keto-compound selected from alpha-ketoacids, alpha-ketoacid esters, alpha-ketoacid amides, glyoxyloyl-derivatized compounds, or mixtures thereof,
(b) at least one surfactant selected from mono-tail or double-tail fatty amidoamines or esteramines, quaternary fatty ammonium compounds, quaternary fatty amidoammonium or esterammonium compounds, fatty imidazolines, complex quaternary ammonium compounds, quaternary ammonium compounds of PEG-ylated carbohydrate derivatives,
(c) at least one thickener selected from guar gum, xanthan gum, carrageenan, pectin, agar, locust bean gum, sclerotium gum, tara gum, gellan gum, konjac gum, arabic gum, alginate, tragacanth gum, karaya gum, diutan gum,
(d) at least one fatty alcohol, and
(e) at least one surfactant different from component (b) and (d),
(f) an aqueous carrier.

The cosmetic composition applied in a step (A) of the methods according to the present invention will be described in more detail in the chapters below.

In step (A) of the methods according to the present invention, the cosmetic composition may be applied to dry hair. The composition may be applied to wet hair. According to embodiments, the composition may be applied to towel-dried hair. According to embodiments, the method may further comprise, prior to step (A), one or more of:
- applying a pre-treatment composition to the hair
- washing the hair
- shampooing the hair and rinsing the shampoo
- towel-drying the hair
- blow-drying the hair

According to embodiments, the hair may be pre-washed, prior to step (A), with shampoo, towel-dried (or blow-dried until hair is towel-dry, i.e. about 20% dry), combed with a wide tooth comb to detangle, and blow-dried until the hair is approximately totally dry.

In step (A) of the method according to the present invention, a brush may be used, if desired, for even application of the composition. The hair may be combed straight and sleek.

In step (B) of the method according to the present invention, the composition may be left on the hair at ambient temperature, conveniently at a temperature in the range of from 18°C to 35°C. The application time (time of contact of the composition with hair) typically will be in the range of from 5 to 60 min, conveniently in the range of from 5 to 40 min, particularly in the range of from 10 to 20 min. When leaving the composition on the hair, typically no heat is applied, and the hair typically is left uncovered.

According to step (C) of the methods according to the present invention, following the application time, the composition is rinsed-off from the hair. According to embodiments, rinsing-off is typically carried out by washing the hair, in particular with plain water (absent shampoo or perfume) for a sufficient time to remove excess components. The rinsing conveniently may be carried out for 1-5 minutes, for example 2-3 minutes.

After rinsing step (C), and prior to heat application step (D), the hair may be again brushed or combed to detangle. According to embodiments, the hair may be at least partially blow-dried prior to heat application step (D), for example until 70-80% dry. According to preferred embodiments, the hair may be blow-dried until essentially totally dry (about 85%-90% dry) prior to heat application step (D), conveniently by using medium heat and high speed. Conveniently, a paddle brush may used during blow-drying.

The application of heat onto hair for the semi-permanent deformation according to step (D) of the methods according to the present invention typically consists of heating hair with a heating device. Suitable heating device temperatures usually are in the range of from 150 to 250°C, for example in the range of from 170 to 230°C, such as in the range of from 185 to 220°C. Particular treatment temperatures often used by stylists are 210°C, or 230°C.

The heating device is suitably is a conventional heating device, typically the heating device is a heating iron, such as a straightening flat iron and a crimping iron. The heating iron usually comprises heating tongs, which are generally metallic heating tongs. When applying heat, the heating device is passed onto hair at least one time, preferably from 5 to 15 times, more preferably from 6 to 10 times.

Following heat application step (D), a post-treatment composition may be applied to the hair, if so desired. The posttreatment composition may be, for example, a conditioning composition.

### 2 Cosmetic compositions for the semi-permanent deformation of keratin fibers, or hair

Cosmetic compositions for the semi-permanent deformation of keratin fibers according to the present invention will be described below. In particular, the compositions according to the present invention are for the semi-permanent deformation of hair, and preferably human hair. Generally, the cosmetic compositions according to the present invention comprise:
(a) at least one keto-compound selected from alpha-ketoacids, alpha-ketoacid esters, alpha-ketoacid amides, glyoxyloyl-derivatized compounds, or mixtures thereof,
(b) at least one surfactant selected from mono-tail or double-tail fatty amidoamines or esteramines, quaternary fatty ammonium compounds, quaternary fatty amidoammonium or esterammonium compounds, fatty imidazolines, complex quaternary ammonium compounds, quaternary ammonium compounds of PEG-ylated carbohydrate derivatives,
(c) at least one thickener selected from guar gum, xanthan gum, carrageenan, pectin, agar, locust bean gum, sclerotium gum, tara gum, gellan gum, konjac gum, arabic gum, alginate, tragacanth gum, karaya gum, diutan gum,
(d) at least one fatty alcohol, and
(e) at least one surfactant different from component (b) and (d),
(f) an aqueous carrier.

According to embodiments, a cosmetic composition according to the present invention essentially consists of, or consists of:
(a) at least one keto-compound selected from alpha-ketoacids, alpha-ketoacid esters, alpha-ketoacid amides, glyoxyloyl-derivatized compounds, or mixtures thereof,
(b) at least one surfactant selected from mono-tail or double-tail fatty amidoamines or esteramines, quaternary fatty ammonium compounds, quaternary fatty amidoammonium or esterammonium compounds, fatty imidazolines, complex quaternary ammonium compounds, quaternary ammonium compounds of PEG-ylated carbohydrate derivatives,
(c) at least one thickener selected from guar gum, xanthan gum, carrageenan, pectin, agar, locust bean gum, sclerotium gum, tara gum, gellan gum, konjac gum, arabic gum, alginate, tragacanth gum, karaya gum, diutan gum,
(d) at least one fatty alcohol, and
(e) at least one surfactant different from component (b) and (d),
(f) a carrier, wherein the carrier is water or a mixture of water and one or more water-soluble polyhydric alcohols,
(g) optionally, one or more pH adjusters selected from (in)organic Brönsted acids and bases,
(h) optionally, one or more additives in a combined amount of less than 10.0 wt%, based on the total weight of the composition.

According to embodiments, the additives are selected from liquid oils, solid fats or waxes, swelling agents, sugar alcohols, preservatives, antioxidants, chelators, electrolytes, pearlescent aids, opacifiers, amino acids/hydrolyzed proteins, and combinations thereof.

### 3 The keto-compounds (a)

The composition comprises, as component (a), at least one keto-compound selected from alpha-ketoacids, alpha-ketoacid esters, alpha-ketoacid amides, glyoxyloyl-derivatized compounds, or mixtures thereof.

Alpha-ketoacids (a) correspond to formula (I) below:

R1-CO-COOH (I)

wherein R1 represents an organic moiety. For the alpha-ketoacids contemplated herein, R1 typically is hydrogen, methyl, formyl, or optionally substituted C1-C6 alkyl.

Alpha-ketoacid esters (a) correspond to formula (II) below:

R1-CO-CO-O-R2 (II)

wherein R1 is as above, and R2 represents an organic moiety. For the alpha-ketoacid esters contemplated herein, R2 typically is hydrogen, methyl, formyl, or optionally substituted C1-C6 alkyl.

Alpha-ketoacid amides (a) correspond to formula (III) below:

R1-CO-CO-NH-R3 (III)

wherein R1 is as above, and R3 represents an organic moiety. For the alpha-ketoacid amides contemplated herein, R3 typically is hydrogen, methyl, formyl, or optionally substituted C1-C6 alkyl.

Glyoxyloyl-derivatized compounds (a) correspond to formula (IV) below:

[R-NH-]-CHO-COOH (IV)

wherein [R-NH-] represents an amino acid moiety bonded via its amino group (or one of its amino groups) to a glyoxyloyl moiety. According to embodiments, [R-NH-] represents a mixture of amino acid moieties bonded via amino groups to glyoxyloyl moieties.

In a preferred embodiment, the at least one keto-compound (a) is present in the composition in the free form, its hydrate forms, its salt forms; more preferably in the free form or its hydrate forms.

In a preferred embodiment, the salt of the at least one keto-compound (a) is selected from the group consisting of the salts of alkali metals and the salts of alkaline-earth metals; preferably the salt of the at least one keto-compound (a) is a sodium salt.

According to embodiments, the at least one keto-compound (a) comprises at least one one alpha-ketoacid selected from glyoxylic acid, pyruvic acid, 2-ketobutyric acid, 2-ketoglutaric acid, or a mixture thereof.

According to embodiments, the at least one keto-compound (a) comprises at least one alpha-ketoacid ester selected from glyoxylic acid ester, pyruvic acid ester, 2-ketobutyric acid ester, 2-ketoglutaric acid ester, or a mixture thereof.

According to embodiments, the at least one keto-compound (a) comprises at least one alpha-ketoacid amide selected from glyoxylic acid amide, pyruvic acid amide, 2-ketobutyric acid amide, 2-ketoglutaric acid amide, or a mixture thereof.

According to embodiments, the at least one keto-compound (a) comprises at least one glyoxyloyl-derivatized compound selected from glyoxyloyl acid, glyoxyloyl acid, ester, glyoxyloyl keratin amino acids, glyoxyloyl carbocysteine, or a mixture thereof.

According to embodiments, the at least one keto-compound (a) is selected from glyoxylic acid, pyruvic acid, 2-ketobutyric acid, 2-ketoglutaric acid, glyoxylic acid ester, glyoxylic acid amide, glyoxyloyl acid, glyoxyloyl keratin amino acids, glyoxyloyl carbocysteine, or a mixture thereof.

According to embodiments, the at least one keto-compound (a) is selected from glyoxylic acid, pyruvic acid, glyoxyloyl keratin amino acids, glyoxyloyl carbocysteine, or a mixture thereof.

According to embodiments, the at least one keto-compound (a) is present in an amount in the range of from 3.0 to 25.0 wt%, typically in the range of from 5.0 wt% to 22.0 wt%, for example in the range of from 10.0 to 19.0 wt%, related to the total weight of the composition.

### 4 The surfactants (b)

The composition comprises, as component (b), at least one at least one surfactant (b) selected from mono-tail or double-tail fatty amidoamines or esteramines, quaternary fatty ammonium compounds, quaternary fatty amidoammonium or esterammonium compounds, fatty imidazolines, complex quaternary ammonium compounds, quaternary ammonium compounds of PEG-ylated carbohydrate derivatives. Cationic surfactants (b) typically are preferred.

According to embodiments, the at least one surfactant (b) is selected from:
(b1) mono-tail or double-tail amidoamines or esteramines according to formula (V):

   [R1-C(O)-A-R2]xN(R3)(2-x)(R4) (V),
(b2) mono-tail or double-tail or triple-tail quaternary ammonium compound according to formula (VI):

   [R1]xN+(R3)(R4)(2-x)(R5) X- (VI),
(b3) mono-tail or double-tail quaternary amidoammonium or esterammonium compound according to formula (VII):

   [R1-C(O)-A-R2]xN+(R3)(R4)(R5)(2-x) X- (VII),
(b4) fatty imidazolines according to formula (VIII):

   R1-[2-imidazoline-1]-R3 (VIII),
(b5) complex quaternary ammonium compounds selected from selected from Quaternium compounds and Polyquaternium compounds,
(b6) quaternary ammonium compounds of an PEG-ylated methyl glucose ether or mixtures thereof.

In the above Formula V, A is -O- in case surfactant (b1) is an esteramine, or -NH- in case the surfactant is an amidoamine. R1 is a saturated fatty chain (fatty alkyl), an unsaturated fatty chain (fatty alkenyl), or a mixture of saturated and/or unsaturated moieties (alkyl and/or alkenyl), wherein at least 60 wt% the moieties of saturated and/or unsaturated fatty chains (fatty alkyl and/or fatty alkenyl). Examples of R1 being a mixture of moieties with at least 60 wt% the moieties of saturated and/or unsaturated fatty chains include fatty acids mixtures derived from naturally occurring oils such as plant oils, for example coconut oil, babassu oil, palm oil. R2 is C2-C3 alkyl. R3 and R4 each are, independently from each other, C1-C3 alkyl or C1-C3 hydroxyalkyl. The nitrogen atom bears a total of three substituents. If one R1-derived fatty chain substituent is present ("mono-tail"), then both substituents R3 and R4 are present (x=1). If two R1-derived fatty chain substituents are present ("double-tail"), then only one substituent R3/R4 is present (x=2). At acidic pH, esteramines or amidoamines are present in protonated form, i.e. the nitrogen atom is quaternized, bearing four substituents, and accordingly bears a positive charge.

In the above Formula VI, the surfactant (b2) is a quaternary ammonium compound. R1 is a saturated fatty chain (fatty alkyl), an unsaturated fatty chain (fatty alkenyl), or a mixture of saturated and/or unsaturated moieties (alkyl and/or alkenyl), wherein at least 60 wt% the moieties of saturated and/or unsaturated fatty chains (fatty alkyl and/or fatty alkenyl). Examples of R1 being a mixture of moieties with at least 60 wt% the moieties of saturated and/or unsaturated fatty chains include, as above, fatty acids mixtures derived from naturally occurring oils such as plant oils, for example coconut oil, babassu oil, palm oil. R3 and R4 each are, independently from each other, C1-C3 alkyl or C1-C3 hydroxyalkyl. R5 is selected from R1, C1-C3 alkyl, C1-C3 hydroxyalkyl, benzyl, [PPG-N+(R3)(R4)(R6) X-], or PROT. The abbreviation PPG in the definition of R5 denotes a propylene glycol derived linker comprising, for example, an average of 2 to 50 moles of propylene glycol, typically an average of 5 to 30 moles of propylene glycol. R6 in the definition of the PPG-ylated moiety is C1-C3 alkyl or C1-C3 hydroxyalkyl (independent from R3 or R4). The abbreviation PROT in the definition of R5 denotes a moiety derived from hydrolyzed protein, linked to R5 for example via an hydroxypropyl linker. The nitrogen atom bears a total of four substituents, and accordingly bears a positive charge. X- denotes a cosmetically acceptable counterion. If one R1-derived fatty chain substituent is present ("mono-tail"), then three substituents R3, R4 and R5 are present (x=1). If two R1-derived fatty chain substituents are present ("double-tail"), then two substituents R3, R5 are present (x=2). If two R1-derived fatty chain substituents are present and additionally R5 is R1, then surfactant (b2) is a "triple-tail" quaternary ammonium compound.

In the above Formula VII, the surfactant (b3) is a quaternary amidoammonium or esterammonium compound. A is -O- in case surfactant (b3) is an esterammonium compound, or -NH-in case the surfactant is an amidoammonium compound. R1 is a saturated fatty chain (fatty alkyl), an unsaturated fatty chain (fatty alkenyl), or a mixture of saturated and/or unsaturated moieties (alkyl and/or alkenyl), wherein at least 60 wt% the moieties of saturated and/or unsaturated fatty chains (fatty alkyl and/or fatty alkenyl). Examples of R1 being a mixture of moieties with at least 60 wt% the moieties of saturated and/or unsaturated fatty chains include, as above, fatty acids mixtures derived from naturally occurring oils such as plant oils, for example coconut oil, babassu oil, palm oil. R2 is C2-C3 alkyl. R3 and R4 each are, independently from each other, C1-C3 alkyl or C1-C3 hydroxyalkyl. R5 is selected from R1, C1-C3 alkyl, C1-C3 hydroxyalkyl, benzyl, [PPG-N+(R3)(R4)(R6) X-], or PROT. The abbreviation PPG in the definition of R5 denotes a propylene glycol derived linker comprising, for example, an average of 2 to 50 moles of propylene glycol, typically an average of 5 to 30 moles of propylene glycol. R6 in the definition of the PPG-ylated moiety is C1-C3 alkyl or C1-C3 hydroxyalkyl (independent from R3 or R4). The abbreviation PROT in the definition of R5 denotes a moiety derived from hydrolyzed protein, linked to R5 for example via an hydroxypropyl linker. The nitrogen atom bears a total of four substituents, and accordingly bears a positive charge. X- denotes a cosmetically acceptable counterion. If one R1-derived fatty chain substituent is present ("mono-tail"), then three substituents selected from R3, R4 and R5 are present (x=1). If two R1-derived fatty chain substituents are present ("double-tail"), then two substituents R3, R4 are present (x=2).

In the above Formula VIII, the surfactant (b4) is a fatty imidazoline. R1 is a saturated fatty chain (fatty alkyl), an unsaturated fatty chain (fatty alkenyl), or a mixture of saturated and/or unsaturated moieties (alkyl and/or alkenyl), wherein at least 60 wt% the moieties of saturated and/or unsaturated fatty chains (fatty alkyl and/or fatty alkenyl). Examples of R1 being a mixture of moieties with at least 60 wt% the moieties of saturated and/or unsaturated fatty chains include, as above, fatty acids mixtures derived from naturally occurring oils such as plant oils, for example coconut oil, babassu oil, palm oil. R3 C1-C3 alkyl or C1-C3 hydroxyalkyl. The nitrogen atom bears a total of three substituents. R1 is attached to the imidazole ring at position 1, i.e. the position between the two imidazole ring nitrogen atoms. R3 is attached to the imidazole ring at position 2, i.e. is attached to one of the two imidazole ring nitrogen atoms

The complex quaternary ammonium compounds (b5) are represented by the so-called Quaternium compounds and Polyquaternium compounds. Particular examples of the complex quaternary ammonium compounds (b5) include, for example, Quaternium-33, Quaternium-8, Quaternium-87, Quaternium-91, Polyquat-10, Polyquat-11, Polyquat-22, Polyquat-37.

The quaternary ammonium compounds of a PEG-ylated methyl glucose ether (b6) include PEG-ylated methyl glucose ethers comprising, for example, an average of 2 to 50 moles of ethylene glycol, typically an average of 5 to 30 moles of ethylene glycol.

According to embodiments, the at least one surfactant (b) comprises a mono-tail amidoamine (b1) selected from arachidamidopropyldimethylamine, arachidamidopropyldiethylamine, arachidamidoethyldiethylamine, arachidamidoethyldimethylamine, behenamidopropyldiethylamine, behenamidopropyldimethylamine, behenamidoethyldiethylamine, behenamidoethyldimethylamine, brassicamidopropyldimethylamine, lauramidopropyldimethylamine, linoleamidopropyldimethylamine, myristamidopropyldimethylamine, oleamidopropyldimethylamine, palmitamidopropyldimethylamine, stearamidopropyldimethylamine, or a combination thereof. For example, the at least one surfactant (b) may comprise arachidamidopropyldimethylamine, arachidamidopropyldiethylamine, behenamidopropyldiethylamine, behenamidopropyldimethylamine, brassicamidopropyldimethylamine, lauramidopropyldimethylamine, myristamidopropyldimethylamine, oleamidopropyldimethylamine, palmitamidopropyldimethylamine, stearamidopropyldimethylamine, or a combination thereof. According to more specific embodiments, the at least one surfactant (b) may comprise brassicamidopropyldimethylamine, lauramidopropyldimethylamine, myristamidopropyldimethylamine, palmitamidopropyldimethylamine, or a combination thereof.

According to embodiments, the at least one surfactant (b) comprises a mono-tail quaternary ammonium compound (b2), wherein the quaternary ammonium cation is selected from arachidtrimonium, cetrimonium, cocotrimonium, steartrimonium, olealkonium, or a combination thereof. For example, the at least one surfactant (b) may comprise a mono-tail quaternary ammonium compound (b2) selected from a cetrimonium, cocotrimonium, or olealkonium compound, or a combination thereof. According to more specific embodiments, the at least one surfactant (b) may comprise cetrimonium methosulfate, cocotrimonium methosulfate, or olealkonium chloride.

According to embodiments, the at least one surfactant (b) comprises a protein-modified quaternary ammonium compound (b2) comprising hydrolyzed protein such as hydrolyzed plant protein. The hydrolyzed plant protein may be, for example, hydrolyzed soy protein or wheat protein. According to more specific embodiments, the at least one surfactant (b) may comprise lauryldimonium hydroxypropyl hydrolyzed soy protein or steardimonium hydroxypropyl hydrolyzed wheat protein.

According to embodiments, the at least one surfactant (b) comprises a double-tail or triple tail quaternary ammonium compound (b2), wherein the quaternary ammonium cation is selected from dicetyldimonium, distearyldimonium, tricetyldimonium , or a combination thereof. According to more specific embodiments, the at least one surfactant (b) may comprise dicetyldimonium chloride, distearyldimonium chloride, or tricetyldimonium chloride.

According to embodiments, the at least one surfactant (b) comprises a mono-tail or double-tail quaternary amidoammonium compound (b3), wherein the quaternary amidoammonium cation is selected from babassuamidopropalkonium, behenamidopropyltrimonium, stearamidopropyltrimonium, behenamidopropyl-PPG-dimonium, dilinoleamidopropyldimonium, or a combination thereof. For example, the at least one surfactant (b) may comprise a quaternary amidoammonium compound (b3) selected from a babassuamidopropalkonium, behenamidopropyl-PPG-dimonium, dilinoleamidopropyldimonium compound, or a combination thereof. According to more specific embodiments, the at least one surfactant (b) may comprise babassuamidopropalkonium chloride, behenamidopropyl-PPG-dimonium chloride, or dilinoleamidopropyldimonium chloride. The PPG-linker of a behenamidopropyl-PPG-dimonium compound may comprise, for example, an average of 2 to 50 moles of propylene glycol, typically an average of 5 to 30 moles of propylene glycol.

According to embodiments, the at least one surfactant (b) comprises a fatty imidazoline (b4). A typical example for a fatty imidazoline (b4) is oleyl hydroxyethyl imidazoline.

According to embodiments, the at least one surfactant (b) comprises a complex quaternary ammonium compound (b5) selected from Quaternium-8, Quaternium-33, and Quaternium-91, Polyquat-10, Polyquat-11, Polyquat-22, Polyquat-37, or a combination thereof. For example, the at least one surfactant (b) may comprises Quaternium-8, Quaternium-33, and/or Quaternium-91.

According to embodiments, the at least one surfactant (b) comprises a quaternary ammonium compound of a PEG-ylated methyl glucose ether (b6). A typical example of a PEG-ylated methyl glucose ether (b6) is a lauryl methyl gluceth-10 hydroxypropyldimonium compound. For example, lauryl methyl gluceth-10 hydroxypropyldimonium chloride is commercially available under the tradename Glucquat^{™} 125 Humectant from Lubrizol.

In cases where the at least one surfactant (b) comprises a compound involving a quaternary nitrogen atom, the counterion conveniently may be selected from cosmetically acceptable anions, such as for example chloride, methosulfate, phosphate.

According to embodiments, the at least one surfactant (b) is present in an amount in the range of from 0.10 to 10.0 wt%, typically in the range of from 0.10 to 5.0 wt%, for example in the range of from 0.50 to 3.0 wt%, related to the total weight of the composition.

### 5 The thickeners (c)

The composition comprises, as component (c), at least one thickener. According to the present invention, the thickener(s) is/are selected from guar gum, xanthan gum, carrageenan, pectin, agar, locust bean gum, sclerotium gum, tara gum, gellan gum, konjac gum, arabic gum, alginate, tragacanth gum, karaya gum, diutan gum, or a combination thereof. The thickeners are added in an amount, or combined amount sufficient to provide the composition with a viscosity so that it can be readily applied to the hair without unduly dripping off the hair and causing mess.

Guar gum is a neutral to slightly anionic polysaccharide composed of galactose and mannose units. It is used as a thickener in cosmetics, food, and pharmaceuticals. Guar gum provides viscosity and stabilizes emulsions.

Xanthan gum is an anionic polysaccharide composed of glucose, mannose, and glucuronic acid units, produced through fermentation by Xanthomonas campestris. It is used in food and cosmetics as a thickener and stabilizer. Xanthan gum provides excellent viscosity and suspension properties.

Carrageenan is an anionic polysaccharide composed of galactose and 3,6-anhydrogalactose, derived from red algae. It is used as a thickener and gelling agent in dairy products, cosmetics, and pharmaceuticals. Carrageenan provides texture and stability.

Pectin is an anionic polysaccharide composed of galacturonic acid units with varying amounts of methyl ester groups. It is used as a gelling agent and thickener in cosmetics and pharmaceuticals. Pectin is also used in jams, jellies, and confectionery.

Agar is a neutral to slightly anionic polysaccharide composed of agarose and agaropectin units, derived from red algae. It is used as a gelling agent in food products and microbiological media, and as a solidifying agent in pharmaceuticals.

Locust bean gum is a neutral galactomannan polysaccharide composed of mannose and galactose units, derived from the seeds of the carob tree. It is used as a thickener and gelling agent in dairy and confectionery products, and in cosmetics for enhancing texture.

Sclerotium gum is a neutral polysaccharide composed of a glucan backbone, produced by the fungus Sclerotium rolfsii. It is used as a thickener and stabilizer in personal care products. Sclerotium gum provides good texture and moisture retention.

Tara gum is a neutral to anionic galactomannan polysaccharide derived from the seeds of the Tara tree, and composed of galactose and mannose units. It is used in food, cosmetics, and pharmaceuticals as a thickener and stabilizer, improving viscosity and texture.

Gellan gum is an anionic polysaccharide composed of glucose, glucuronic acid, and rhamnose units, produced through fermentation by Sphingomonas elodea. It is used as a gelling agent and thickener in cosmetics, but also in food products like jellies, gelatin substitutes, and beverages.

Konjac gum is a neutral to slightly anionic polysaccharide composed of glucomannan, a polymer of glucose and mannose. It is used in cosmetics for thickening, gelling and emulsifying, and in food (e.g., konjac noodles, vegan meat substitutes).

Arabic gum is an anionic complex polysaccharide composed of galactose, rhamnose, arabinose, and glucuronic acid units. It is used as a thickener, emulsifier and stabilizer in cosmetics, confectionery, and beverages. Arabic gum provides hydration and film-forming properties.

Alginate is an anionic polysaccharide composed of mannuronic acid and guluronic acid units, derived from brown algae. It is used as a thickener, gelling agent, and stabilizer in cosmetics, pharmaceuticals, and food products.

Tragacanth gum is a neutral to slightly anionic complex polysaccharide composed of arabinose, galactose, and rhamnose units, obtained from the sap of the tragacanth plant. It is used as a thickener and emulsifier in cosmetics, pharmaceuticals, and food products. Tragacanth gum provides suspension and gelation properties.

Karaya gum is an anionic polysaccharide composed of galactose, rhamnose, and mannose units, derived from the Sterculia tree. It is used as a thickener and emulsifier in cosmetics and pharmaceuticals. Karaya gum provides viscosity, moisture retention, and is used in adhesives.

Diutan gum is an anionic polysaccharide composed by six sugar units of d- glucose, d-glucuronic acid, d-glucose (with 2 I-rhamnose in the side chain) and I-rhamnoses, forming a linear backbone with a repeating side chain. Diutan gum is a water-soluble polysaccharide, which may be produced by microbial fermentation, particularly from Sphingomonas. Diutan gum is used as a thickener and rheology modifier. Diutan gum is commercially available under the tradename Kelco-Care^{™} Diutan Gum from CP Kelco France or Lubrizol.

According to embodiments, the thickener (c) is selected from agar, locust bean gum, tara gum, gellan gum, konjac gum, arabic gum, alginate, tragacanth gum, karaya gum, or a combination thereof. For example, the thickener (c) may be selected from locust bean gum, tara gum, konjac gum, alginate, tragacanth gum, karaya gum, or a combination thereof. According to more specific embodiments, the thickener (c) may be selected from locust bean gum, konjac gum, alginate, tragacanth gum, karaya gum, or a combination thereof

According to embodiments, the thickener(s) (c) have a weight average molecular weight in the range of from 0.50 million Daltons to 8.0 million Daltons, for example in the range of from 1.20 million Daltons 5.80 million Daltons.

According to embodiments, the at least one thickener (c) is present in an amount in the range of from 0.010 to 1.0 wt%, typically in the range of from 0.010 to 0.50 wt%, for example in the range of from 0.010 to 0.30 wt%, related to the total weight of the composition.

### 6 The fatty alcohols (d)

The composition comprises, as component (d), at least one fatty alcohol. The term "fatty alcohol" as used herein denotes a non-alkoxylated, saturated or unsaturated, linear or branched, alcohol comprising a linear carbon chain of from 14 to 30 carbon atoms.

According to embodiments, the at least one fatty alcohol (d) is selected from the group consisting of linear and/or branched C14 to C30 fatty alcohols; in particular from the group consisting of linear C14 to C30 fatty alcohols. Specific examples of fatty alcohols suitable in the present invention comprise cetyl alcohol, stearyl alcohol, cetearyl alcohol and behenyl alcohol. One specific exemplary fatty alcohol (d) is cetearyl alcohol.

According to embodiments, the at least one fatty alcohol (d) is present in an amount in the range of from 2.50 to 20.0 wt%, typically in the range of from 3.0 to 15.0 wt%, for example in the range of from 4.0 to 10.0 wt%, related to the total weight of the composition.

### 7 The surfactants (e) [different from components (b) and (d)]

The composition comprises, as component (e), at least one non-ionic surfactant different from fatty alcohol (d), and different from surfactant (b).

According to embodiments, the at least one surfactant (e) may be selected from non-ionic, cationic, amphoteric surfactants, or a combination thereof. According to more specific, and typically preferred embodiments, the at least one surfactant (e) is selected from one or more non-ionic surfactants.

According to embodiments, the at least one non-ionic surfactant (e) is selected from the group consisting of non-ionic surfactants comprising one or more polyethylene glycol (PEG) chains (PEG-ylated compounds). Examples of PEG-ylated non-ionic surfactants include: PEG alkyl ethers, PEG fatty acid esters, PEG castor oil, PEG hydrogenated castor oil, PEG fatty amides and their monoethanolamine and diethanolamine derivatives and PEG-ylated fatty amines. More preferably, the at least one non-ionic surfactant is selected from the group consisting of PEG C8 to C30 alkyl ethers; even more preferably from the group consisting of PEG-ylated C8 to C30 alkyl ethers having at least 2 ethylene glycol units, or at least 5 ethylene glycol units; yet more preferably from the group consisting of PEG-ylated C8 to C30 alkyl ethers having from 10 to 50 ethylene glycol units; most preferably from the group consisting of PEG-ylated C8 to C30 alkyl ethers having from 15 to 30 ethylene glycol units. Particular examples of the at least one non-ionic surfactant (e) are ceteareth-25 and steareth-20. One specific exemplary non-ionic surfactant (e) is Steareth-20.

According to embodiments, the at least one surfactant (e), typically non-ionic surfactant (e), is present in amount in the range of from 0.20 to 5.0 wt%, typically in the range of from 0.50 to 4.0 wt%, for example in the range of from 1.0 to 2.50 wt%, related to the total weight of the composition.

### 8 The aqueous carrier (f)

The composition comprises, as component (f), a cosmetically acceptable carrier or solvent. According to embodiments, the composition may comprise water as the main cosmetically acceptable carrier (f), and one or more water-soluble polyhydric alcohols as secondary cosmetically acceptable carriers. According to embodiments, the cosmetically acceptable carrier (f) of the composition may consist essentially of, or consist of water.

Water-soluble polyhydric alcohols may have two or more hydroxyl groups in the molecule. Polyhydric alcohols for example may be selected from:
- dihydric alcohols such as ethylene glycol, 1,2-propanediol (propylene glycol), 1,3-propanediol, 1,2-pentanediol; further, trimethylene glycol, 1,2-butylene glycol, 1,3-butylene glycol, 1,4-butylene glycol, tetramethylene glycol, 2,3- butylene glycol, pentamethylene glycol, 2-butene-1,4-diol, hexylene glycol, octylene glycol
- trihydric alcohols such as glycerol, trimethylol propane, 1,2,6-hexanetriol and the like
- tetrahydric alcohols such as penthaerythritol
- pentahydric alcohols such as xylytol, etc.
- hexahydric alcohols such as sorbitol, mannitol
- polyhydric alcohol polymers such as diethylene glycol, dipropylene glycol, polyethylene glycol, polypropylene glycol, tetraethylene glycol, diglycerol, polyethylene glycol, triglycerol, tetraglycerol, polyglycerol; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether
- dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether
- dihydric alcohol etheresters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate and propylene glycol monophenyl ether acetate

According to embodiments, the composition may comprise water as the main cosmetically acceptable carrier (f), and one or more water-soluble polyhydric alcohols as secondary cosmetically acceptable carriers. According to embodiments, the water-soluble polyhydric alcohols may be selected from dihydric and trihydric alcohols. According to typical embodiments, the carrier may be a mixture of water and one or more alcohols selected from ethylene glycol, propylene glycol, 1,3-propanediol, butanediol, pentanediol, trimethylene glycol, tetramethylene glycol, glycerol, trimethylol propane. According to more specific embodiments, the carrier (f) may be a mixture of water and glycerol, or a mixture of water and propylene glycol (1,2-propanediol). One specific exemplary aqueous carrier (f) is a mixture of water and glycerol.

When the aqueous carrier (f) is a mixture of water and one or more water-soluble polyhydric alcohols, water typically is present in an amount of 50.0 wt%, preferably of 55.0 wt%, more preferably of 60.0 wt%, related to the total weight of the composition, and the remaining amount of the cosmetically acceptable carrier consists of the one or more water-soluble polyhydric alcohols. According to embodiments, the water may be present in an amount in the range of from 50.0 to 85.0 wt%, suitably in the range of from 55.0 to 80.0 wt%, for example in the range of from 60.0 to 75.0 wt%, related to the total weight of the composition, and the remaining amount of the cosmetically acceptable carrier consists of the one water-soluble polyhydric alcohol.

Termed differently, when the aqueous carrier (f) is a mixture of water and one or more water-soluble polyhydric alcohols, the weight ratio of the water to the one or more water-soluble polyhydric alcohols typically is within a range of from 10.0 to 30.0, for example within a range of from 12.0 to 20.0.

According to other embodiments, the composition may comprise water as the sole cosmetically acceptable carrier (f), in which case the composition is essentially free of, or free of water-soluble polyhydric alcohols. According to embodiments, the water may be present in an amount of 55.0 wt% or more, suitably in an amount of 60.0 wt% or more, for example in an amount of 65.0 wt% or more, related to the total weight of the composition. According to embodiments, the water may be present in an amount in the range of from 55.0 to 90.0 wt%, suitably in the range of from 60.0 to 85.0 wt%, for example in the range of from 65.0 to 80.0 wt%, related to the total weight of the composition.

Irrespective of its exact nature, the cosmetically acceptable carrier may present in an amount of 55.0 wt% or more, suitably in an amount of 60.0 wt% or more, for example in an amount of 65.0 wt% or more, related to the total weight of the composition. According to embodiments, the cosmetically acceptable carrier may be present in an amount in the range of from 55.0 to 90.0 wt%, suitably in the range of from 60.0 to 85.0 wt%, for example in the range of from 65.0 to 80.0 wt%, related to the total weight of the composition.

### 9 The optional pH adjusters (g), and pH of the composition

According to embodiments, the composition has a pH of 4.0 or less, typically in the range of from 1.0 to 3.50, for example in the range of from 1.50 to 3.0.

In acidic environments, amidoamine or esteramine surfactants (b1) are present in protonated form, i.e. the nitrogen atom is quaternized, and accordingly surfactants (b1) are positive at acidic conditions. Surfactants (b2) to (b6) all are quaternized ammonium compounds anyway, i.e. cationic compounds. Accordingly, at acidic conditions component (b) is a cationic surfactant irrespective of the exact nature of the surfactant. Having surfactant (b) present in the form of a cationic compound typically is preferable.

The pH of the composition optionally may be adjusted using pH adjusting agents, including acidifying and/or basifying agents. Acidifying agents generally are organic or inorganic Brönsted acids. Basifying agents generally are organic or inorganic Brönsted bases. The acidifying agents may be selected for example, from formic acid, C2-C6 mono-, di- and tri-carboxylic (hydroxy) acids, phosphoric acid, sulfuric acid, sulfonic acid, carbonic acid. Salts of the above acids may be suitable as pH adjusters and/or buffering agents. The basifying agents may be selected from the group consisting of mineral alkaline agents, organic alkaline agents, and hydride alkaline agents. For example, suitable mineral alkaline agents may be selected from the group consisting of sodium hydroxide and potassium hydroxide; preferably the mineral alkaline agent is sodium hydroxide.

### 10 Optional additives (h)

According to embodiments, the composition may comprise one or more optional additives (h). Suitable additives (h) for the compositions according to the present invention comprise in particular liquid oils, solid fats or waxes, swelling agents, sugar alcohols, preservatives, antioxidants, chelators, electrolytes, pearlescent aids, opacifiers, amino acids/hydrolyzed proteins, and combinations thereof.

According to embodiments, the additives may be present in the compositions according to the present invention in a combined amount of 10.0 wt% or less. Typically, the additives may be present in the compositions according to the present invention in a combined amount of 8.0 wt% or less. For example, the additives may be present in the compositions according to the present invention in a combined amount of 6.0 wt% or less. Alternatively, the compositions according to the present invention may be essentially free of, or free of additives.

### 10.1 Liquid oils

According to embodiments, the composition comprises at least one liquid oil.

For example, the liquid oil may be selected from the group consisting of avocado oil, tsubaki oil, turtle oil, macadamia nuts oil, corn oil, mink oil, olive oil, rape seed oil, yolk oil, sesame oil, parsic oil, wheat germ oil, sasanqua oil, castor oil, linseed oil, safflower oil, cotton seed oil, perilla oil, soybean oil, peanut oil, tea seed oil, kaya oil, rice bran oil, Chinese tung oil, Japanese tung oil, jojoba oil, germ oil, triglycerides, trioctanoic acid glyceride, triisopalmitic acid glyceride, liquid lanolin, hexyl laurate, liquid hydrocarbon esters such as fluid paraffin, mineral oil, isododecane, fatty acid oils, squalene, ester oils such as cetyl octanoate and isopropyl myristate.

If present, the at least one liquid oil is present in an amount in the range of from 0.1 to 5.0 wt%, related to the total weight of the composition. Alternatively, the composition may be essentially free of, or free of a liquid oil. The absence of a liquid oil may help avoiding generating a significant amount of fume, when the hair is heated, for example by using a flat iron.

### 10.2 Solid fats and waxes

In a preferred embodiment, the composition comprises at least one solid fat or wax.

For example, the solid fat or wax may be selected from the group consisting of cacao fat, coconut oil, horse fat, hardened coconut fat, palm oil, tallow, sheep fat, hardened tallow, palm kernel oil, lard, ox bone fat, wood wax kernel oil, hardened castor oil; lanolin, kapok wax, lanolin acetate, isopropyl lanolin fatty acid, reduced lanolin, hard lanolin, POE lanolin alcohol ether, POE lanolin alcohol acetate, POE cholesterol ether, lanolin fatty acid polyethylene glycol, POE hydrogenated lanolin alcohol ether; hydrocarbons, non-volatile hydrocarbons and solid hydrocarbon esters such as solid paraffin, vaseline, ozocerite, pristan, ceresin, petrolatum, microcrystalline wax; phospholipides, e.g., lecithins or ceramides, beeswax, apple wax, candelilla wax, cotton wax, carnauba wax, bayberry wax, insect wax, whale wax, montan wax, rice bran wax, kapok wax, cane wax, jojoba wax, and shellac wax. The wax is not a fatty alcohol.

If present, the at least one solid fat or wax is present in an amount in the range of from 0.1 to 5.0 wt%, related to the total weight of the composition. Alternatively, the composition may be essentially free of, or free of a solid fat or wax. The absence of a solid fat or wax may help avoiding generating a significant amount of fume, when the hair is heated, for example by using a flat iron.

### 10.3 Swelling agents

In a preferred embodiment, the composition comprises at least one swelling agent. So-called swelling agents and penetrating substances may be added to the hair shaping composition, examples being urea, melamine, ethers, e. g. dipropylene glycol monomethyl ether, 2-pyrrolidon, imidazolidin-2-one, 1-methyl-2-pyrrolidone; alkali or ammonium thiocyanate, polyvalent alcohols, isopropanol; preferably the swelling agent and penetrating substance is urea.

If present, the swelling agent is present in an amount in the range of from 0.1 to 5.0 wt.-%, related to the total weight of the composition. Alternatively, the composition may be essentially free of, or free of swelling agents.

### 10.4 Other additives optionally present

The composition may further comprise other additives, in particular other additives selected from sugar alcohols, preservatives, antioxidants, chelators, electrolytes, pearlescent aids, opacifiers, amino acids/hydrolyzed proteins, and combinations thereof.

Suitable sugar alcohols comprise, for example, sorbitol, maltitol, maltotriose, mannitol, sucrase, erythritol, glucose, fructose, starch sugar, maltose, xylytose, starch sugar reduced alcohol. Suitable preservatives comprise, for example, benzyl alcohol and imidazolidinyl urea. Suitable antioxidants include, for example, tocopheroles. Suitable chelators comprise, for example, disodium ethylenediamine tetraacetate, ethylenediamine-N,N'-disuccinic acid and its salts. Suitable electrolytes comprise, for example, sodium chloride, potassium chloride, ammonium chloride, sodium sulfate, potassium sulfate and ammonium sulfate salts. Suitable pearlescent aids comprise, for example, ethylene glycol distearate and Mica. Suitable opacifiers comprise, for example, kaolin and titanium oxide. Suitable amino acids and/or hydrolysed proteins comprise, for example, keratin amino acids, and hydrolyzed plant proteins such as hydrolyzed soy proteins or hydrolyzed wheat proteins.

If present, each of the sugar alcohols, electrolytes, and pearlescent aids typically are present in an amount in the range of from 0.10 to 5.0 wt%; each of the preservatives, antioxidants, chelators, opacifiers, and/or amino acids and hydrolysed proteins is present in an amount in the range of from 0.01 to 3.0 wt% of, related to the total weight of the composition.

### 11 Additives preferably absent from the compositions

### 11.1 Additional surfactants (essentially free of, or free of)

According to embodiments, the composition may comprise at least one additional surfactant, other than surfactants (b), (d) and (e), albeit in a low amount, e.g., in an amount in the range of from 0.1 to 5.0 wt%. Additional surfactants may be selected from anionic surfactants, and amphoteric surfactants.

Typically, however, the composition is essentially free of, or free of an additional surfactant, other than surfactants (b), (d) and (e). According to embodiments, the composition is essentially free of, or free of additional surfactants, in particular anionic surfactant and/or amphoteric surfactants.

### 11.2 Additional thickeners / rheology modifiers (essentially free of, or free of)

The composition typically is essentially free of, or free of additional thickeners and/or rheology modifiers, other than the thickeners according to component (c).

If present, additional thickeners and/or rheology modifiers may be selected from hydroxypropyl guar, scleroglucan, methylcellulose, ethyl cellulose, hydroxyethyl cellulose, carboxymethyl cellulose, hydroxypropylmethyl cellulose, microcrystalline cellulose, hydroxybutylmethyl cellulose, hydroxypropyl cellulose (Klucel), hydroxyethyl ethyl cellulose, cetyl hydroxyethyl cellulose (Natrosol Plus 330), N- vinylpyrollidone (Povidone), Acrylates/Ceteth-20 Itaconate Copolymer (Structure 3001), hydroxypropyl starch phosphate, PEG-ylated urethanes or polycarbamyl polyglycol ester such as PEG-150/Decyl/SMDI copolymer (Aculyn 44), PEG-150/Stearyl/SMDI copolymer (Aculyn 46), trihydroxystearin (Thixcin), acrylates copolymer (Aculyn 33) or hydrophobically modified acrylate copolymers (such as Acrylates I Steareth-20 Methacrylate Copolymer, for example Aculyn 22), acrylates/steareth-20 methacrylate crosspolymer (Aculyn 88), acrylates/vinyl neodecanoate crosspolymer (Aculyn 38), acrylates/beheneth-25 methacrylate copolymer (Aculyn 28), acrylates/C10-30 alkyl acrylate crosspolymer (Carbopol ETD 2020), and non-ionic amphophilic polymers comprising at least one fatty chain and at least one hydrophilic unit selected from polyether urethanes comprising at least one fatty chain.

### 11.3 Cationic polymers (essentially free of, or free of)

In a preferred embodiment, the composition is essentially free of, or free of a cationic polymer, except for complex quaternary ammonium compounds (b5), if present.

If present, the cationic polymers may be selected from the group consisting of cationic homopolymers, cationic copolymers, cationic polysaccharides, cationic silicon polymers comprising quaternary nitrogen groups, and mixtures thereof.

### 11.4 Reducing agents (essentially free of, or free of)

In a preferred embodiment, the composition is essentially free of, or free of a reducing agent, particularly a reducing agent comprising a mercapto-functional group. Reducing agents are compounds, which are suitable for reducing the disulfide bonds of the hair. Reducing agents are usually present in cosmetic compositions for the permanent deformation of keratin fibers. For example, the reducing agents may be selected from the group consisting of cysteamine, cysteine, thioglycolic acid and their salts thereof.

### 11.5 Alkalizing agents (essentially free of, or free of)

In a preferred embodiment, the composition is essentially free of, or free of an alkalizing agent, other than pH adjusting agents. Alkalizing agents are usually present in cosmetic compositions for the permanent deformation of keratin fibers. In case an alkalizing agent should be present in the composition according to the invention, it may be selected from the group consisting of ammonia and monoethanolamine.

### 11.6 Colouring agents (essentially free of, or free of)

In a preferred embodiment, the composition is essentially free of, or free of a colouring agent. Colouring agents typically are selected from the group consisting of direct dyes, pigments and oxidation dye precursor (including bases and couplers).

### 11.7 Silicones (essentially free of, or free of)

The composition according to the present invention is essentially free of, or free of silicones, such as for example silicone copolyols, such as dimethicone copolyols, e.g., polyether siloxane-modified polymers, such as polypropylene glycol, polyethylene glycol modified polydimethylsiloxane, polyalkyl siloxanes, polyaryl siloxanes, polyalkylaryl siloxanes, polyether siloxane copolymers, dimethylpolysiloxane containing terminal hydroxyl groups, methylphenyl polysiloxane containing terminal hydroxyl groups and mixtures thereof, low molecular weight oligomeric polydimethylsiloxane or cyclic polydimethylsiloxane, polyether siloxane copolymers, dimethicone, amodimethicone, dimethiconol, dimethicone copolyol and cyclodimethicone.

According to preferred embodiments, the composition according to the present invention is free of silicones.

### 11.8 UV filters and sunscreens

The compositions according to the present invention are essentially free of UV filters and sunscreens such as, for example, p-methoxy cinnamic acid isoamylester, lipophile cinnamic acid esters, salicylic acid esters, 4-amino benzoic acid derivatives or hydrophilic sulfonic acid derivatives of benzophenones or 3-benzyliden campher.

According to embodiments, the compositions according to the present invention are free of UV filters and sunscreens.

### 11.9 Perfume oils and fragrances

The compositions according to the present invention are essentially free of perfume oils and fragrances. According to embodiments, the compositions according to the present invention are free of perfume oils and fragrances.

### 12 Weight ratio fatty alcohol (d) : surfactant (e)

According to embodiments, surfactant (e) is at least one non-ionic surfactant (e), and the composition comprises fatty alcohol (d) to non-ionic surfactant (e) in a weight ratio in the range of from 20.0 to 2.50 (10.0:0.50 to 10.0:4.0), typically in the range of from 10.0 to 2.860 (10.0:1.0 to 10.0:3.5); for example in the range of from 6.67 to 3.33 (10.0:1.5 to 10.0:3.0).

### 13 Gel network system

According to embodiments, the at least one fatty alcohol (d) and the surfactant (e) are comprised in the composition, in part or all, in a gel network system. According to embodiments, surfactant (e) is at least one non-ionic surfactant (e), and the at least one fatty alcohol (d) and the non-ionic surfactant (e) are comprised in the composition, in part or all, in a gel network system.

According to embodiments, the composition comprises a total amount of the fatty alcohol (d) and the non-ionic surfactant (e) as described herein above in the range of from 2.70 to 25.0 wt%, typically in the range of from 3.50 to 19.0 wt%, more preferably in the range of from 5.0 to 12.50 wt%, related to the total weight of the composition.

Typically, the formation of the gel network involves heating a dispersion of the at least one fatty alcohol in water with the surfactant to a temperature above 80 °C. During the mixing process, the fatty alcohol melts, allowing the surfactant to partition into the fatty alcohol droplets. The surfactant brings water along with it into the at least one fatty alcohol. This changes the isotropic fatty alcohol drops into liquid crystalline phase drops. When the mixture is cooled below the chain melt temperature the liquid crystal phase is converted into a solid crystalline gel network. The gel network may particularly contribute to reduce, or even eliminate, the odour of compounds comprising a mercapto-functional group.

According to embodiments, from 50.0 to 100.0 wt%, more preferably from 70.0 to 100.0 wt%, even more preferably from 90.0 to 100.0 wt%, most preferably 100.0 wt% of the fatty alcohol (d) is comprised within the gel network. According to embodiments, from 50.0 to 100.0 wt%, more preferably from 70.0 to 100.0 wt%, even more preferably from 90.0 to 100.0 wt%, most preferably 100.0 wt% of the surfactant (e) is comprised within the gel network.

### 14 Viscosity

In a preferred embodiment, the composition has a viscosity at 25°C in the range of from 100 to 2,000 mPa.s, preferably in the range of from 200 to 1,500 mPa.s, more preferably in the range of from 500 to 1,000 mPa.s.

In a preferred embodiment, the viscosity is determined as follows: Viscosity measurements are carried out on a controlled stress rheometer of AR500, AR1000 or AR2000 type manufactured by TA Instruments, or equivalent instrument. A 6 cm flat acrylic cross hatched parallel plate geometry (TA item 518600.901) and a stainless-steel cross hatched base plate (TA item 570011.001) are used. The rheometer is prepared for flow measurements as per standard manufacturer procedure. The parallel plate geometry gap is set to 1,000 microns. The flow procedure is programmed to the rheometer with the following conditions: continuous stress ramp 0.1-300 Pa over 2 minutes at 25°C, including 250 measurement points in linear mode. The product is loaded into the geometry as per standard procedure. Shear stress value at 10 sec¹ shear rate is obtained from the shear stress vs. shear rate curve, and the corresponding viscosity is calculated by dividing the obtained shear stress by 10.

### 15 Application form

The composition according to the present invention is a rinse-off composition. By "rinse-off' is meant a composition, which is rinsed off after application onto hair, particularly before flat-ironing hair.

The inventors have shown that, after application, the composition generates minimal fume when hair heated, for example by using a flat iron. This is advantageous, particularly by comparison with conventional cosmetic compositions used for the semi-permanent deformation of hair. Indeed, conventional cosmetic compositions may generate a significant amount of fume, when hair is heated, for example by using a flat iron. This is inconvenient for the hairdresser and/or the user, as it may irritate the eyes and mucous membranes, and generate an unwanted/unpleasant smell. This is particularly inconvenient in hair salons, whether several hairdressers may perform the treatment at the same time.

### 16 Use

In a third aspect, the presently claimed invention relates to the use of the composition described above, for the semi-permanent deformation of hair.

### 17 Advantages

The presently claimed invention offers one or more of the following advantages:
- The composition provides a superior semi-permanent shaping performance;
- The composition provides hair manageability and hair conditioning;
- The composition does not impart any unpleasant stinging and itching sensation;
- The composition does not damage hair;
- The composition generates a minimal amount of fume upon application;
- The composition provides satisfactory consistency, rinsability, combability after wash (before flat ironing), combability with product, hair feel before flat ironing, hair feel after blow drying and/or straightening iron use;
- The composition is easy to manufacture because it contains only very few components; and
- The composition is not deposited on the hair.

### EXAMPLES

The presently claimed invention is illustrated in detail by non-restrictive working examples which follow. More particularly, the test methods specified hereinafter are part of the general disclosure of the application and are not restricted to the specific working examples.

Keratin Treatments (KT) are popular cosmetic products containing Glyoxylic acid (GA) as active ingredient designed to enhance the styling of human hair. However, due to its potential release of formaldehyde during the high-temperature ironing process (up to 230°C) that is essential for best product performance, the use of substance is regulated in several countries. For the study, airborne formaldehyde was collected during product application on mannequin heads within a controlled model environment. This approach simulated product application in hair salons and allowed for the quantitative measurement of formaldehyde.

The formaldehyde exposure study is designed to estimate potential consumer and hairdresser exposures to Formaldehyde released from Keratin Treatments during product and heat application and compares the levels to the exposure limit of 0.12 mg/m³ (120 µg/m³) and 0.1 mg/m³ (100 µg/m³) given by ACGIH (8-hour workplace limit; 2017) and the WHO (indoor air quality limit; 2010), respectively, for product safety assessment.

### EXAMPLE 1 Formaldehyde Exposure Study

Two cosmetic composition samples are prepared for a Formaldehyde Exposure Study, i.e. Keratin treatment composition according to sample 1, and a negative control sample (placebo). The two compositions comprise the ingredients shown in table 1.

### 1.1 Formulations

**Table 1: Formulations**

| | Sample 1 Keratin Treatment @ 210/230°C | negative control (placebo) |
|---|---|---|
| | [wt%] | [wt%] |
| Glyoxylic acid^{A} | 12.00 | 0.00 |
| Cetearyl alcohol | 5.75 | 5.75 |
| Glycerol | 5.99 | 5.99 |
| Glucquat 125⁸ | 1.50 | 1.50 |
| Steareth-20 | 0.48 | 0.48 |
| Sodium hydroxide | 1.20 | 0.00 |
| Diutan gum^{C} | 0.12 | 0.12 |
| Water | Q.s. 100 | Q.s. 100 |
| pH | 2.20 | 6.40 |

| | | |
|---|---|---|
| A Glyoxo HighPure 50, available from Weylchem B Glucquat^{™} 125 Humectant, available from Lubrizol C Kelco-Care^{™} Diutan Gum, available from Lubrizol | | |

### 1.2 Preparation of the cosmetic composition

The formation of the gel network involves heating a dispersion of the fatty alcohol in water with the non-ionic surfactant to a temperature above 80°C. During the mixing process, the fatty alcohol melts, allowing the surfactant to partition into the fatty alcohol droplets. The surfactant brings water along with it into the fatty alcohol. This changes the isotropic fatty alcohol drops into liquid crystalline phase drops. When the mixture is cooled below the chain melt temperature, the liquid crystal phase is converted into a solid crystalline gel network. Then, the other materials are added to the mixture obtained.

### 1.3 Product Application

The Keratin treatment (KT) as well as the corresponding negative control (KT w/o glyoxylic acid) are applied according to the procedure outlined below. Before the actual treatment the mannequin head is pre-washed once with shampoo.

**Table 2: Product application steps**

| | |
|---|---|
| Preparation: Hair Drying | • comb hair with a wide-tooth comb to detangle |
| | • blow-dry with medium heat and speed until hair is towel-dry (20% dry) |
| | • continue blow-drying with high speed until hair is approximately 80% dry |
| Keratin Treatment Application | • part hair into 4 sections using a cross-shaped parting |
| | • apply product strand by strand (strands about 2 cm wide), maintaining a distance of 0.5 cm from the roots |
| | • use a brush for even application and comb hair straight and sleek |
| | • let the product sit for 20 minutes without heat or covering |
| Rinsing | • rinse hair thoroughly for about 3 min until all residue is removed |
| | • comb hair to detangle |
| Hair Drying and Detangling | • blow-dry with medium heat and high-speed using a paddle brush until hair is 100% dry |
| | • comb again if necessary |
| Strand Preparation | • part hair into 4 sections (cross-shaped), then divide into thin strands (ca. 0.5 cm wide) |
| Flat Ironing | • straighten hair strand by strand with a flat iron at 210°C/230°C |
| | • pass the flat iron 8 times per strand (approx. 18 seconds per stroke) |

### 1.4 Determination of Formaldehyde Exposure

The Keratin Treatments with glyoxylic acid are carried out at two flat iron temperatures, i.e. 210°C and 230°C, for better understanding the influence of the flat iron temperature on the release of formaldehyde. A negative control sample, Keratin Treatment without glyoxylic acid (Placebo), is included in the study to estimate the non-glyoxylic acid driven product and hair contribution to the overall formaldehyde level in the room. The flat iron temperature of the negative control is 210°C.

The placebo negative controls, and the keratin treatments with glyoxylic acid each are carried out in duplicate (placebo: samples #1, #2; KT 210°C: samples #3, #4; KT 230°C: samples #5, #6).

The actual amounts of composition applied are indicated in Table 3. The application steps, and the amount of time required for each step are indicated in Table 4.

**Table 3: amounts of composition applied**

| placebo (210°C) | | treatment/210° C | | treatment/230°C | |
|---|---|---|---|---|---|
| #1 | #2 | #3 | #4 | #5 | #6 |

| **amount of composition applied / [g]** | | | | | |
|---|---|---|---|---|---|
| 70 | 73 | 80 | 82 | 82 | 80 |

**Table 4: application steps, and timing**

| | | placebo (210°C) | | treatment/210°C | | treatment/230°C | |
|---|---|---|---|---|---|---|---|
| | | #1 | #2 | #3 | #4 | #5 | #6 |
| | | **time [min]** | | | | | |
| 1 | hair preparation incl. application | 32 | 32 | 31 | 30 | 30 | 31 |
| 2 | waiting time after appl. and rinsing | 27 | 26 | 25 | 26 | 26 | 25 |
| 3 | blow dry, detangle, hair prep.* | 10 | 14 | 21 | 17 | 14 | 17 |
| 4 | flat ironing | 32 | 40 | 51 | 42 | 45 | 36 |
| 5 | time overall | 101 | 112 | 128 | 115 | 115 | 109 |
| *incl. time needed by the stylist for putting on face mask, for samples #3 to #6 | | | | | | | |

### 1.5 Results and discussion

Formaldehyde concentrations obtained for negative control samples #1 and #2 across all three measurement points ranged from 2.8 to 4.6 µg/m3. Formaldehyde concentrations found for the product application at 210°C are slightly increased compared to the placebo formulation, ranging from 5.6 to 8.3 µg/m3. For the product application at 210°C, formaldehyde concentrations further increased, ranging from 6.4 to 13.3 µg/m3.

Formaldehyde levels of placebo samples #1 and #2 were approximately 50% of the levels measured for the 210°C KT formulation containing 16% Glyoxylic acid (samples #3 and #4).

Measured room blanks all are below 1 µg/m3 (prior to and between product applications).

Among the measurement locations tested, the mannequin head location has the highest exposure levels, followed by the stylist location. Overall, the levels do not vary significantly over the three measurement locations tested.

**Table 5: Results**

| | | placebo (210°C) | | treatment/210°C | | treatment/230°C | |
|---|---|---|---|---|---|---|---|
| | | #1 | #2 | #3 | #4 | #5 | #6 |
| | | **formaldehyde concentration*** / **[µg/m3]** | | | | | |
| 1 | room | 3.1 | 2.8 | 5.6 | 8.0 | 8.2 | 6.4 |
| 2 | mannequin head | 3.8 | 3.0 | 8.3 | 7.9 | 11.3 | 13.3 |
| 3 | stylist | 4.6 | 3.8 | 7.7 | 7.7 | 9.6 | 8.1 |
| 4 | blank measurement (room) | <1 | <1 | <1 | <1 | <1 | <1 |
| * determined at the end of the application | | | | | | | |

### 2 Formaldehyde Exposure Test - methodology

Formaldehyde testing is carried out by a test method according to ISO 16000-3, 2022-09 (Determination of formaldehyde and other carbonyl compounds in indoor and test chambers air - active sampling method), except for modification of the formaldehyde detection used. The method used in the present invention uses tandem mass spectroscopy (MS/MS) for the formaldehyde detection, which is more precise and selective than the UV detection used in ISO 16000-3, 2022-09.

### 2.1 Measurement principle

In accordance with NIOSH Manual of Analytical Methods (Formaldehyde: Method 2016, Issue 2, 15 March 2003, Page 2-7; NIOSH Manual of Analytical Methods (NMAM), 4th Addition), air sampling is performed with calibrated pumps by passing room air through a cartridge containing 2,4-Dinitrophenylhydrazone (DNPH) as a derivatization active. In presence of Formaldehyde, the derivative Formaldehyde-2,4-DNPH is formed, eluted with organic solvent and subjected to high performance liquid chromatographic (HPLC) analysis. Detection and external quantification are performed by tandem mass spectrometry (MS/MS) operating in the multiple reaction monitoring (MRM) mode. All measurements are executed in duplicate, and results reported in µg/m3 taking the flow of the pumps (1 L/min.) and the air sampling period into account.

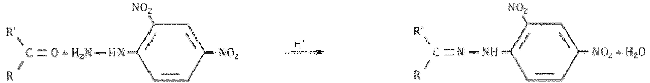

The study is not conducted according to GLP, but the work is performed according to written work instructions and fulfils internal laboratory standards ensuring reliability of the results.

### 2.2 Experimental design

The experimental investigation for characterizing inhalation exposure is conducted using a realistic simulation test. A small chamber (15.0 m3), corresponding to small-scale hair straightening zones in a hair dresser salon is used as a model environment (see tables 6, 7). To cover worst-case surrounding conditions in which consumer or professional may use the products, no ventilation (i.e. no air-conditioning) and no exchange of room air is carried out during exposure measurements. The product application is performed by a professional Stylist using an artificial mannequin head as study object, following the steps given in table 8.

Two individual applications are carried out for each:
- negative control (to understand non-glyoxylic acid driven formaldehyde background levels)
- product (keratin treatment), with heat treatment @210°C
- product (keratin treatment), with heat treatment @230°C
   to account for possible variations in the application procedure and aerosol release process.

All airborne formaldehyde is accumulated over the times given in table 8 and quantified after completion of all stages of product application. After sampling for an individual product application, the exposure chamber is ventilated for at least 10 min at an air exchange rate of about 442 m3/h. For each individual product test, a formaldehyde free mannequin test head is pre-washed and blow dried. Formaldehyde concentration is measured directly in the breathing zone of the stylist and in addition at the breathing zone of the mannequin head and in the far field zone on the floor.

**Table 6: Environmental conditions for air sampling**

| | |
|---|---|
| Room Volume | 15 m3 |
| Room Temperature | Ambient, between 17-25°C |
| Room Humidity | Ambient, between 30-58% rH |
| Floor Covering | Laminate |
| Walls | Glas |
| Ceiling | Metal |
| Air Exchange | No air-conditioning or ventilation |
| Pump flow rate | 1 L/min |

**Table 7: Sampling area**

| | |
|---|---|
| Mannequin Head | Cartridge taped to head with open end to the side of the nose |
| Stylist* | Cartridge clipped onto lab coat with open end facing to the breathing area about 20 cm from the nose |
| Test environment | Cartridge at a distance of ~1m from the mannequin head at a height of about 50-70 cm from floor |
| * for safety reasons, stylists wear a face mask during the straightening process when KT formulations are used | |

**Table 8: Application steps and sampling points**

| | | approx. time | room | mannequin head | stylist |
|---|---|---|---|---|---|
| 1 | wetting, blow drying and product application | 30 min | | | |
| 2 | waiting time after application, rinsing and combing | 25 min | | | |
| 3 | hair blow drying | 15 min | | | |
| 4 | flat ironing | 45-60 min | | | |
| 5 | sampling points (@ end of application) | | x | x | x |
| | background measured prior to new application | | x | | |

Before and during the measurements, special attention is given to potential formaldehyde background levels, which could arise from nearby sources or inadequate ventilation following each release. To address this, background measurements (n=2) are conducted prior to the measurement campaign and before each new exposure measurement (after ventilation).

### 2.3 Materials and Equipment

**Table 9: Tools**

| | |
|---|---|
| Amount product applied per test leg | 60-80 grams |
| Blow dryer | GHD Helios HE001 |
| Flat iron | Babyliss PRO Series 3000 Elipsis 3100 |
| Mannequin heads | Doll heads formaldehyde free, Euro-natural hair, 28 cm hair length, light brown 5/0 hair color |
| Pumps | Escort Elf Pump; Zefon International, Cat. No. A4-20248, calibrated; Flow rate 1L/min |
| Cartridge Type | LpDNPH S-Series; Supelco, Cat. No. 21014 |
| Face Mask | Series 9000, Moldex-Metric AG & CO. KG; EN 136:1998 CL2 |

### 2.4 Calculation and Reporting

The mass of released Formaldehyde is determined from the calibration graph, and if necessary corrected for background levels (blank value). The final results are reported in µg/m3. $Formaldehyde \left[μg/m3\right] = \frac{Formaldehyde \left[μg\right] - Blank \left[μg\right]}{Air Volume Sampled \left[m3\right]}$

### EXAMPLE 2

Keratin treatment compositions of samples 2 and 3 shown in table 10 are prepared.

**Table 10: Formulations**

| | **sample 2** | **sample 3** |
|---|---|---|
| pyruvic acid | 14.00 | 0.00 |
| alpha keto glutaric acid | 0.00 | 18.00 |
| Cetearyl alcohol | 8.00 | 8.00 |
| Glycerol | 4.99 | 4.99 |
| Stearamidopropyltrimonium | 2.00 | 2.00 |
| PEG hydrogenated castor oil | 4.00 | 4.00 |
| Sodium hydroxide | 1.20 | 1.20 |
| Locust Bean Gum | 0.50 | 0.00 |
| Tara Gum | 0.00 | 1.00 |
| Water | Q.s. 100 | Q.s. 100 |
| pH | 2.50 | 2.60 |

When tested for formaldehyde emissions as decribed in Example 1, the formaldehyde exposure levels obtained with samples 2 and 3 are well below the regulatory exposure limits given by ACGIH (8-hour workplace limit; 2017) and the WHO (indoor air quality limit; 2010), respectively.

### Embodiments

Illustrative embodiments of the presently claimed invention are listed below, but do not restrict the presently claimed invention.

Variations and modifications of the invention and further embodiments thereof, in addition to those described herein, will become apparent to those skilled in the art from the full contents of this document. The subject matter herein contains information, exemplification and guidance that can be adapted to the practice of this invention in its various embodiments and equivalents thereof. It is intended that the appended claims cover all such variations, modifications, embodiments and equivalents.

While the presently claimed invention has been described in terms of its specific embodiments, certain modifications and equivalents will be apparent to those skilled in the art and are intended to be included within the scope of the presently claimed invention.

The following embodiments further describe the present invention.
1 A method for the semi-permanent deformation of hair, the method comprising the steps of:
   (A) applying a cosmetic composition onto hair,
   (B) leaving the composition on the hair for a suitable application time,
   (C) rinsing off the composition from the hair, and
   (D) applying heat onto the hair using a heating device,
   wherein the cosmetic composition comprises:
   (a) at least one keto-compound selected from alpha-ketoacids, alpha-ketoacid esters, alpha-ketoacid amides, glyoxyloyl-derivatized compounds, or mixtures thereof,
   (b) at least one surfactant selected from mono-tail or double-tail fatty amidoamines or esteramines, quaternary fatty ammonium compounds, quaternary fatty amidoammonium or esterammonium compounds, fatty imidazolines, complex quaternary ammonium compounds, quaternary ammonium compounds of PEG-ylated carbohydrate derivatives,
   (c) at least one thickener selected from guar gum, xanthan gum, carrageenan, pectin, agar, locust bean gum, sclerotium gum, tara gum, gellan gum, konjac gum, arabic gum, alginate, tragacanth gum, karaya gum, diutan gum,
   (d) at least one fatty alcohol, and
   (e) at least one surfactant different from component (b) and (d),
   (f) an aqueous carrier.
2 The method according to embodiment 1, further comprising the step of shampooing the hair prior to step (A).
3 The method according to embodiment 1 or 2, wherein the application time in step (B) is 5 to 40 minutes, preferably 10 to 20 minutes.
4 The method according to any one of the preceding embodiments, wherein a rinsing medium used in step (C) is water.
5 The method according to any one of the preceding embodiments, wherein in step (D) a temperature of the heating device is in the range of from 150 to 250°C, preferably in the range of from 170 to 230°C, more preferably in the range of from 185 to 220°C.
6 The method according to any one of the preceding embodiments, wherein the heating device used in step (D) is a heating iron, preferably the heating iron is selected from the group consisting of a straightening flat iron or a crimping iron.
7 The method according to any one of the preceding embodiments, wherein the heating device used in step (D) is a heating iron, and wherein up to 15 passes, in particular 5 to 12 passes, for example 6 to 10 passes of the heating iron are applied onto the hair.
8 The method according to any one of the preceding embodiments, further comprising after step (C) and prior to step (D) at least partially drying the hair using a blow dryer.
9 The method according to any one of the preceding embodiments, further comprising after step (C) and prior to step (D) essentially totally drying the hair using a blow dryer.
10 The method according to any one of the preceding embodiments, wherein the cosmetic composition essentially consists of, or consists of:
   (a) at least one keto-compound selected from alpha-ketoacids, optionally in the form of a salt or hydrate, or alpha-ketoacid esters, or alpha-ketoacid amides, or glyoxyloyl-derivatized compounds, or mixtures thereof,
   (b) at least one surfactant selected from mono-tail or double-tail fatty amidoamines or esteramines, quaternary fatty ammonium compounds, quaternary fatty amidoammonium or esterammonium compounds, fatty imidazolines, complex quaternary ammonium compounds, quaternary ammonium compounds of PEG-ylated carbohydrate derivatives,
   (c) at least one thickener selected from guar gum, xanthan gum, carrageenan, pectin, agar, locust bean gum, sclerotium gum, tara gum, gellan gum, konjac gum, arabic gum, alginate, tragacanth gum, karaya gum, diutan gum,
   (d) at least one fatty alcohol, and
   (e) at least one surfactant different from component (b) and (d),
   (f) a carrier, wherein the carrier is water or a mixture of water and one or more water-soluble polyhydric alcohols,
   (g) optionally, one or more pH adjusters selected from (in)organic Brönsted acids and bases,
   (h) optionally, one or more additives in a combined amount of 10.0 wt% or less, based on the total weight of the composition.
11 The method according to any one of the preceding embodiments, wherein the at least one keto-compound (a) comprises at least one one alpha-ketoacid selected from glyoxylic acid, pyruvic acid, 2-ketobutyric acid, 2-ketoglutaric acid, or a mixture thereof.
12 The method according to any one of the preceding embodiments, wherein the at least one keto-compound (a) comprises at least one alpha-ketoacid ester selected from glyoxylic acid ester, pyruvic acid ester, 2-ketobutyric acid ester, 2-ketoglutaric acid ester, or a mixture thereof.
13 The method according to any one of the preceding embodiments, wherein the at least one keto-compound (a) comprises at least one alpha-ketoacid amide selected from glyoxylic acid amide, pyruvic acid amide, 2-ketobutyric acid amide, 2-ketoglutaric acid amide, or a mixture thereof.
14 The method according to any one of the preceding embodiments, wherein the at least one keto-compound (a) comprises at least one glyoxyloyl-derivatized compound selected from glyoxyloyl acid, glyoxyloyl acid ester, glyoxyloyl keratin amino acids, glyoxyloyl carbocysteine, or a mixture thereof.
15 The method according to any one of the preceding embodiments, wherein the at least one keto-compound (a) is selected from glyoxylic acid, pyruvic acid, 2-ketobutyric acid, 2-ketoglutaric acid, glyoxylic acid ester, glyoxylic acid amide, glyoxyloyl acid, glyoxyloyl keratin amino acids, glyoxyloyl carbocysteine, or a mixture thereof.
16 The method according to any one of the preceding embodiments, wherein the at least one keto-compound (a) is selected from glyoxylic acid, pyruvic acid, glyoxyloyl keratin amino acids, glyoxyloyl carbocysteine, or a mixture thereof.
17 The method according to any one of the preceding embodiments, wherein the at least one surfactant (b) is selected from:
   (b1) mono-tail or double-tail amidoamines or esteramines according to formula (V):

      [R1-C(O)-A-R2]xN(R3)(2-x)(R4) (V),
   (b2) mono-tail or double-tail or triple-tail quaternary ammonium compounds according to formula (VI):

      [R1]xN+(R3)(R4)(2-x)(R5) X- (VI),
   (b3) mono-tail or double-tail quaternary amidoammonium or esterammonium compounds according to formula (VII):

      [R1-C(O)-A-R2]xN+(R3)(R4)(R5)(2-x) X- (VII),
   (b4) fatty imidazolines according to formula (VIII):

      R1-[2-imidazoline-1]-R3 (VIII),
   (b5) complex quaternary ammonium compounds selected from Quaternium compounds and Polyquaternium compounds,
   (b6) quaternary ammonium compounds of an PEG-ylated methyl glucose ether,
   or mixtures thereof,
   wherein A is -O- or -NH-,
   R1 is fatty alk(en)yl, or a mixture of alk(en)yl comprising at least 60 wt% fatty alk(en)yl,
   R2 is C2-C3 alkyl,
   R3, R4 and R6 each are, independently from each other, C1-C3 alkyl or C1-C3 hydroxyalkyl,
   R5 is selected from R1, C1-C3 alkyl, C1-C3 hydroxyalkyl, benzyl, [PPG-N+(R3)(R4)(R6) X-], or PROT,
   PROT is a moiety derived from hydrolyzed protein,
   PPG is a propylene glycol derived linker,
   X- is a cosmetically acceptable counterion, and
   x is 1 or 2.
18 The method according to any one of the preceding embodiments, wherein the at least one surfactant (b) comprises a mono-tail amidoamine (b1) selected from arachidamidopropyldimethylamine, arachidamidopropyldiethylamine, arachidamidoethyldiethylamine, arachidamidoethyldimethylamine, behenamidopropyldiethylamine, behenamidopropyldimethylamine, behenamidoethyldiethylamine, behenamidoethyldimethylamine, brassicamidopropyldimethylamine, lauramidopropyldimethylamine, linoleamidopropyldimethylamine, myristamidopropyldimethylamine, oleamidopropyldimethylamine, palmitamidopropyldimethylamine, stearamidopropyldimethylamine.
19 The method according to any one of the preceding embodiments, wherein the at least one surfactant (b) comprises a mono-tail quaternary ammonium compound (b2), wherein the quaternary ammonium cation is selected from arachidtrimonium, cetrimonium, cocotrimonium, steartrimonium, olealkonium.
20 The method according to any one of the preceding embodiments, wherein the at least one surfactant (b) comprises a protein-modified quaternary ammonium compound (b2), wherein the hydrolyzed protein of PROT of R5 is hydrolyzed plant protein.
21 The method according to any one of the preceding embodiments, wherein the at least one surfactant (b) comprises a mono-tail protein-modified quaternary ammonium compound (b2) selected from lauryldimonium hydroxypropyl hydrolyzed soy protein and steardimonium hydroxypropyl hydrolyzed wheat protein.
22 The method according to any one of the preceding embodiments, wherein the at least one surfactant (b) comprises a double-tail or triple tail quaternary ammonium compound (b2), wherein the quaternary ammonium cation is selected from dicetyldimonium, distearyldimonium, tricetyldimonium.
23 The method according to any one of the preceding embodiments, wherein the at least one surfactant (b) comprises a mono-tail or double-tail quaternary amidoammonium compound (b3), wherein the quaternary ammonium cation is selected from babassuamidopropalkonium, behenamidopropyltrimonium, stearamidopropyltrimonium, behenamidopropyl-PPG-dimonium, dilinoleamidopropyldimonium.
24 The method according to any one of the preceding embodiments, wherein the at least one surfactant (b) comprises a fatty imidazoline (b4), wherein the fatty imidazoline is oleyl hydroxyethyl imidazoline.
25 The method according to any one of the preceding embodiments, wherein the at least one surfactant (b) comprises a complex quaternary ammonium compound (b5) selected from Quaternium-8, Quaternium-33, and Quaternium-91, Polyquat-10, Polyquat-11, Polyquat-22, Polyquat-37.
26 The method according to any one of the preceding embodiments, wherein the at least one surfactant (b) comprises a quaternary ammonium compound of a PEG-ylated methyl glucose ether (b6), wherein the quaternary ammonium cation is lauryl methyl gluceth-10 hydroxypropyldimonium.
27 The method according to any one of the preceding embodiments, wherein the at least one surfactant (b) comprises a quaternary ammonium compound, and wherein the counterion is selected from chloride, methosulfate, phosphate.
28 The method according to any one of the preceding embodiments, wherein theat least one thickener (c) is selected from agar, locust bean gum, tara gum, gellan gum, konjac gum, arabic gum, alginate, tragacanth gum, karaya gum.
29 The method according to any one of the preceding embodiments, wherein the at least one thickener (c) is selected from locust bean gum, tara gum, konjac gum, alginate, tragacanth gum, karaya gum.
30 The method according to any one of the preceding embodiments, wherein the at least one thickener (c) is selected from locust bean gum, konjac gum, alginate, tragacanth gum, karaya gum.
31 The method according to any one of the preceding embodiments, wherein the at least one fatty alcohol (d) is selected from the group consisting of linear or branched C14 to C30 fatty alcohols, more preferably from the group consisting of linear C14 to C30 fatty alcohols, even more preferably from the group consisting of cetyl alcohol, stearyl alcohol, cetearyl alcohol and behenyl alcohol.
32 The method according to any one of the preceding embodiments, wherein the at least one surfactant (e) is selected from non-ionic, cationic, amphoteric surfactants, or a combination thereof.
33 The method according to any one of the preceding embodiments, wherein the at least one surfactant (e) is a non-ionic surfactant selected from the group consisting of PEG-ylated C8 to C30 alkyl ethers, preferably from the group consisting of PEG-ylated C8 to C30 alkyl ethers having at least 2 ethylene glycol units, or at least 5 ethylene glycol units, more preferably from the group consisting of PEG-ylated C8 to C30 alkyl ethers having from 10 to 50 ethylene glycol units, preferably from the group consisting of PEG-ylated C8 to C30 alkyl ethers having from 15 to 30 ethylene glycol units, more preferably from the group consisting of ceteareth-25 and steareth-20.
34 The method according to any one of the preceding embodiments, wherein the carrier (f) is a mixture of water and one or more dihydric and trihydric alcohols, in particular wherein the carrier is a mixture of water and one or more alcohols selected from ethylene glycol, propylene glycol, 1,3-propanediol, butanediol, pentanediol, trimethylene glycol, tetramethylene glycol, glycerol, trimethylol propane.
35 The method according to any one of the preceding embodiments, wherein the carrier (f) is a mixture of water with propylene glycol or glycerol.
36 The method according to any one of the preceding embodiments, wherein the carrier (f) is a mixture of water and glycerol.
37 The method according to any one of embodiments 1 to 33, wherein the carrier (f) is water.
38 The method according to any one of the preceding embodiments, wherein the composition comprises one or more pH adjuster (g), wherein the pH adjuster(s) are selected from formic acid, C2-C6 mono-, di- and tri-carboxylic (hydroxy) acids, phosphoric acid, sulfuric acid, sulfonic acid, carbonic acid, or salts thereof, hydroxides, in particular sodium hydroxide.
39 The method according to any one of the preceding embodiments, wherein the composition comprises one or more additives (h), wherein the additives are selected from liquid oils, solid fats or waxes, swelling agents, sugar alcohols, preservatives, antioxidants, chelators, electrolytes, pearlescent aids, opacifiers, amino acids/hydrolyzed proteins.
40 The method according to any one of the preceding embodiments, wherein the composition is essentially free of, or free of silicones.
41 The method according to any one of the preceding embodiments, wherein the composition is essentially free of, or free of colouring agents.
42 The method according to any one of the preceding embodiments, wherein the composition is essentially free of, or free of perfume oils, fragrances, UV filters and sunscreens.
43 The method according to any one of the preceding embodiments, wherein:
   (a) the at least one keto-compound (a) is present in an amount in the range of from 3.0 to 18.0 wt%, preferably in the range of from 5.0 wt% to 17.0 wt%, more preferably in the range of from 10.0 to 16.0 wt%, related to the total weight of the composition,
   (b) the at least one quaternary ammonium compound of an PEG-ylated methyl glucose ether is present in an amount in the range of from 0.1 to 10.0 wt%, more preferably in the range of from 0.1 to 5.0 wt%, even more preferably in the range of from 0.5 to 3.0 wt%, related to the total weight of the composition,
   (c) the at least one thickener is present in an amount in the range of from 0.01 to 1.0 wt%, preferably in the range of from 0.01 to 0.5 wt%, more preferably in the range of from 0.01 to 0.3 wt%, related to the total weight of the composition,
   (d) the at least one fatty alcohol is present in an amount in the range of from 2.5 to 20.0 wt%, preferably in the range of from 3.0 to 15.0 wt%, more preferably in the range of from 4.0 to 10.0 wt%, related to the total weight of the composition, and/or
   (e) the at least one non-ionic surfactant is present in an amount in the range of from 0.2 to 5.0 wt%, more preferably in the range of from 0.5 to 4.0 wt%, even more preferably in the range of from 1.0 to 2.5 wt%, related to the total weight of the composition.
44 The method according to any one of the preceding embodiments, wherein the composition has a pH of 4.0 or less, preferably in the range of from 1.0 to 3.50, more preferably in the range of from 1.50 to 3.0.
45 The method according to any one of the preceding embodiments, wherein the composition has a viscosity at 25°C in the range of from 100 to 2,000 mPa.s, preferably in the range of from 200 to 1,500 mPa.s, more preferably in the range of from 500 to 1,000 mPa.s determined according to the method of the description.
46 The method according to any one of the preceding embodiments, wherein the composition comprises the component (d) to the component (e) in a weight ratio in the range of from 10.0:0.5 to 10.0:4.0, more preferably in the range of from 10.0:1.0 to 10.0:3.5, even more preferably in the range of from 10.0:1.5 to 10.0:3.0.
47 The method according to any one of the preceding embodiments, wherein the component (d) and the component (e) are comprised in the composition, in part or all, in a gel network system.
48 The method according to any one of the preceding embodiments, wherein the formaldehyde exposure, as determined by the Formaldehyde Exposure Test, in the room, and at the location of the stylist, and at the location of the mannequin head, is 80.0 µg/m3 or less, in particular 50.0 µg/m3 or less.
49 The method according to any one of the preceding embodiments, wherein for a heat treatment at 210°C or 230°C the formaldehyde exposure, as determined by the Formaldehyde Exposure Test, in the room, and at the location of the stylist, and at the location of the mannequin head, is 40.0 µg/m3 or less, in particular 30.0 µg/m3 or less.
50 The method according to any one of the preceding embodiments, wherein for a heat treatment at 210°C or 230°C the formaldehyde exposure, as determined by the Formaldehyde Exposure Test, in the room, and at the location of the stylist, and at the location of the mannequin head, is 25.0 µg/m3 or less, in particular 20.0 µg/m3 or less.
51 A cosmetic composition for the semi-permanent deformation of hair, comprising:
   (a) at least one keto-compound selected from alpha-ketoacids, alpha-ketoacid esters, alpha-ketoacid amides, glyoxyloyl-derivatized compounds, or mixtures thereof,
   (b) at least one cationic surfactant selected from mono-tail or double-tail fatty amidoamines or esteramines, quaternary fatty ammonium compounds, quaternary fatty amidoammonium or esterammonium compounds, fatty imidazolines, complex quaternary ammonium compounds, quaternary ammonium compounds of PEG-ylated carbohydrate derivatives,
   (c) at least one thickener selected from guar gum, xanthan gum, carrageenan, pectin, agar, locust bean gum, sclerotium gum, tara gum, gellan gum, konjac gum, arabic gum, alginate, tragacanth gum, karaya gum, diutan gum,
   (d) at least one fatty alcohol, and
   (e) at least one surfactant different from component (b) and (d),
   (f) an aqueous carrier.
52 The cosmetic composition according to embodiment 51, essentially consisting of, or consisting of:
   (a) at least one keto-compound selected from alpha-ketoacids, optionally in the form of a salt or hydrate, or alpha-ketoacid esters, or alpha-ketoacid amides, or glyoxyloyl-derivatized compounds, or mixtures thereof,
   (b) at least one surfactant selected from mono-tail or double-tail fatty amidoamines or esteramines, quaternary fatty ammonium compounds, quaternary fatty amidoammonium or esterammonium compounds, fatty imidazolines, complex quaternary ammonium compounds, quaternary ammonium compounds of PEG-ylated carbohydrate derivatives,
   (c) at least one thickener selected from guar gum, xanthan gum, carrageenan, pectin, agar, locust bean gum, sclerotium gum, tara gum, gellan gum, konjac gum, arabic gum, alginate, tragacanth gum, karaya gum, diutan gum,
   (d) at least one fatty alcohol, and
   (e) at least one surfactant different from component (b) and (d),
   (f) a carrier, wherein the carrier is water or a mixture of water and one or more water-soluble polyhydric alcohols,
   (g) optionally, one or more pH adjusters selected from (in)organic Brönsted acids and bases,
   (h) optionally, one or more additives in a combined amount of 10.0 wt% or less, based on the total weight of the composition.
53 The cosmetic composition according to embodiment 51 or 52, wherein the at least one keto-compound (a) comprises at least one one alpha-ketoacid selected from glyoxylic acid, pyruvic acid, 2-ketobutyric acid, 2-ketoglutaric acid, or a mixture thereof.
54 The cosmetic composition according to any one of embodiments 51 to 53, wherein the at least one keto-compound (a) comprises at least one alpha-ketoacid ester selected from glyoxylic acid ester, pyruvic acid ester, 2-ketobutyric acid ester, 2-ketoglutaric acid ester, or a mixture thereof.
55 The cosmetic composition according to any one of embodiments 51 to 54, wherein the at least one keto-compound (a) comprises at least one alpha-ketoacid amide selected from glyoxylic acid amide, pyruvic acid amide, 2-ketobutyric acid amide, 2-ketoglutaric acid amide, or a mixture thereof.
56 The cosmetic composition according to any one of embodiments 51 to 55, wherein the at least one keto-compound (a) comprises at least one glyoxyloyl-derivatized compound selected from glyoxyloyl acid, glyoxyloyl acid ester, glyoxyloyl keratin amino acids, glyoxyloyl carbocysteine, or a mixture thereof.
57 The cosmetic composition according to any one of embodiments 51 to 56, wherein the at least one keto-compound (a) is selected from glyoxylic acid, pyruvic acid, 2-ketobutyric acid, 2-ketoglutaric acid, glyoxylic acid ester, glyoxylic acid amide, glyoxyloyl acid, glyoxyloyl keratin amino acids, glyoxyloyl carbocysteine, or a mixture thereof.
58 The cosmetic composition according to any one of embodiments 51 to 57, wherein the at least one keto-compound (a) is selected from glyoxylic acid, pyruvic acid, glyoxyloyl keratin amino acids, glyoxyloyl carbocysteine, or a mixture thereof.
59 The cosmetic composition according to any one of embodiments 51 to 58, wherein the at least one surfactant (b) is selected from:
   (b1) mono-tail or double-tail amidoamines or esteramines according to formula (V):

      [R1-C(O)-A-R2]xN(R3)(2-x)(R4) (V),
   (b2) mono-tail or double-tail or triple-tail quaternary ammonium compounds according to formula (VI):

      [R1]xN+(R3)(R4)(2-x)(R5) X- (VI),
   (b3) mono-tail or double-tail quaternary amidoammonium or esterammonium compounds according to formula (VII):

      [R1-C(O)-A-R2]xN+(R3)(R4)(R5)(2-x) X- (VII),
   (b4) fatty imidazolines according to formula (VIII):

      R1-[2-imidazoline-1]-R3 (VIII),
   (b5) complex quaternary ammonium compounds selected from Quaternium compounds and Polyquaternium compounds,
   (b6) quaternary ammonium compounds of an PEG-ylated methyl glucose ether,
   or mixtures thereof,
   wherein A is -O- or -NH-,
   R1 is fatty alk(en)yl, or a mixture of alk(en)yl comprising at least 60 wt% fatty alk(en)yl,
   R2 is C2-C3 alkyl,
   R3, R4 and R6 each are, independently from each other, C1-C3 alkyl or C1-C3 hydroxyalkyl,
   R5 is selected from R1, C1-C3 alkyl, C1-C3 hydroxyalkyl, benzyl, [PPG-N+(R3)(R4)(R6) X-], or PROT,
   PROT is a moiety derived from hydrolyzed protein,
   PPG is a propylene glycol derived linker,
   X- is a cosmetically acceptable counterion, and
   x is 1 or 2.
60 The cosmetic composition according to any one of embodiments 51 to 59, wherein the at least one surfactant (b) comprises a mono-tail amidoamine (b1) selected from arachidamidopropyldimethylamine, arachidamidopropyldiethylamine, arachidamidoethyldiethylamine, arachidamidoethyldimethylamine, behenamidopropyldiethylamine, behenamidopropyldimethylamine, behenamidoethyldiethylamine, behenamidoethyldimethylamine, brassicamidopropyldimethylamine, lauramidopropyldimethylamine, linoleamidopropyldimethylamine, myristamidopropyldimethylamine, oleamidopropyldimethylamine, palmitamidopropyldimethylamine, stearamidopropyldimethylamine.
61 The cosmetic composition according to any one of embodiments 51 to 60, wherein the at least one surfactant (b) comprises a mono-tail quaternary ammonium compound (b2), wherein the quaternary ammonium cation is selected from arachidtrimonium, cetrimonium, cocotrimonium, steartrimonium, olealkonium.
62 The cosmetic composition according to any one of embodiments 51 to 61, wherein the at least one surfactant (b) comprises a protein-modified quaternary ammonium compound (b2), wherein the hydrolyzed protein of PROT of R5 is hydrolyzed plant protein.
63 The cosmetic composition according to any one of embodiments 51 to 62, wherein the at least one surfactant (b) comprises a mono-tail protein-modified quaternary ammonium compound (b2) selected from lauryldimonium hydroxypropyl hydrolyzed soy protein and steardimonium hydroxypropyl hydrolyzed wheat protein.
64 The cosmetic composition according to any one of embodiments 51 to 63, wherein the at least one surfactant (b) comprises a double-tail or triple tail quaternary ammonium compound (b2), wherein the quaternary ammonium cation is selected from dicetyldimonium, distearyldimonium, tricetyldimonium.
65 The cosmetic composition according to any one of embodiments 51 to 64, wherein the at least one surfactant (b) comprises a mono-tail or double-tail quaternary amidoammonium compound (b3), wherein the quaternary ammonium cation is selected from babassuamidopropalkonium, behenamidopropyltrimonium, stearamidopropyltrimonium, behenamidopropyl-PPG-dimonium, dilinoleamidopropyldimonium.
66 The cosmetic composition according to any one of embodiments 51 to 65, wherein the at least one surfactant (b) comprises a fatty imidazoline (b4), wherein the fatty imidazoline is oleyl hydroxyethyl imidazoline.
67 The cosmetic composition according to any one of embodiments 51 to 66, wherein the at least one surfactant (b) comprises a complex quaternary ammonium compound (b5) selected from Quaternium-8, Quaternium-33, and Quaternium-91, Polyquat-10, Polyquat-11, Polyquat-22, Polyquat-37.
68 The cosmetic composition according to any one of embodiments 51 to 67, wherein the at least one surfactant (b) comprises a quaternary ammonium compound of a PEG-ylated methyl glucose ether (b6), wherein the quaternary ammonium cation is lauryl methyl gluceth-10 hydroxypropyldimonium.
69 The cosmetic composition according to any one of embodiments 51 to 68, wherein the at least one surfactant (b) comprises a quaternary ammonium compound, and wherein the counterion is selected from chloride, methosulfate, phosphate.
70 The cosmetic composition according to any one of embodiments 51 to 69, wherein theat least one thickener (c) is selected from agar, locust bean gum, tara gum, gellan gum, konjac gum, arabic gum, alginate, tragacanth gum, karaya gum.
71 The cosmetic composition according to any one of embodiments 51 to 70, wherein the at least one thickener (c) is selected from locust bean gum, tara gum, konjac gum, alginate, tragacanth gum, karaya gum.
72 The cosmetic composition according to any one of embodiments 51 to 71, wherein the at least one thickener (c) is selected from locust bean gum, konjac gum, alginate, tragacanth gum, karaya gum.
73 The cosmetic composition according to any one of embodiments 51 to 72, wherein the at least one fatty alcohol (d) is selected from the group consisting of linear or branched C14 to C30 fatty alcohols, more preferably from the group consisting of linear C14 to C30 fatty alcohols, even more preferably from the group consisting of cetyl alcohol, stearyl alcohol, cetearyl alcohol and behenyl alcohol.
74 The cosmetic composition according to any one of embodiments 51 to 73, wherein the at least one surfactant (e) is selected from non-ionic, cationic, amphoteric surfactants, or a combination thereof.
75 The cosmetic composition according to any one of embodiments 51 to 74, wherein the at least one surfactant (e) is a non-ionic surfactant selected from the group consisting of PEG-ylated C8 to C30 alkyl ethers, preferably from the group consisting of PEG-ylated C8 to C30 alkyl ethers having at least 2 ethylene glycol units, or at least 5 ethylene glycol units, more preferably from the group consisting of PEG-ylated C8 to C30 alkyl ethers having from 10 to 50 ethylene glycol units, preferably from the group consisting of PEG-ylated C8 to C30 alkyl ethers having from 15 to 30 ethylene glycol units, more preferably from the group consisting of ceteareth-25 and steareth-20.
76 The cosmetic composition according to any one of embodiments 51 to 75, wherein the carrier (f) is a mixture of water and one or more dihydric and trihydric alcohols, in particular wherein the carrier is a mixture of water and one or more alcohols selected from ethylene glycol, propylene glycol, 1,3-propanediol, butanediol, pentanediol, trimethylene glycol, tetramethylene glycol, glycerol, trimethylol propane.
77 The cosmetic composition according to any one of embodiments 51 to 76, wherein the carrier (f) is a mixture of water with propylene glycol or glycerol.
78 The cosmetic composition according to any one of embodiments 51 to 77, wherein the carrier (f) is a mixture of water and glycerol.
79 The cosmetic composition according to any one of embodiments 51 to 75, wherein the carrier (f) is water.
80 The cosmetic composition according to any one of embodiments 51 to 79, wherein the composition comprises one or more pH adjuster (g), wherein the pH adjuster(s) are selected from formic acid, C2-C6 mono-, di- and tri-carboxylic (hydroxy) acids, phosphoric acid, sulfuric acid, sulfonic acid, carbonic acid, or salts thereof, hydroxides, in particular sodium hydroxide.
81 The cosmetic composition according to any one of embodiments 51 to 80, wherein the composition comprises one or more additives (h), wherein the additives are selected from liquid oils, solid fats or waxes, swelling agents, sugar alcohols, preservatives, antioxidants, chelators, electrolytes, pearlescent aids, opacifiers, amino acids/hydrolyzed proteins.
82 The cosmetic composition according to any one of embodiments 51 to 81, wherein the composition is essentially free of, or free of silicones.
83 The cosmetic composition according to any one of embodiments 51 to 82, wherein the composition is essentially free of, or free of colouring agents.
84 The cosmetic composition according to any one of embodiments 51 to 83, wherein the composition is essentially free of, or free of perfume oils, fragrances, UV filters and sunscreens.
85 The cosmetic composition according to any one of embodiments 51 to 84, wherein:
   (a) the at least one keto-compound (a) is present in an amount in the range of from 3.0 to 18.0 wt%, preferably in the range of from 5.0 wt% to 17.0 wt%, more preferably in the range of from 10.0 to 16.0 wt%, related to the total weight of the composition,
   (b) the at least one quaternary ammonium compound of an PEG-ylated methyl glucose ether is present in an amount in the range of from 0.1 to 10.0 wt%, more preferably in the range of from 0.1 to 5.0 wt%, even more preferably in the range of from 0.5 to 3.0 wt%, related to the total weight of the composition,
   (c) the at least one thickener is present in an amount in the range of from 0.01 to 1.0 wt%, preferably in the range of from 0.01 to 0.5 wt%, more preferably in the range of from 0.01 to 0.3 wt%, related to the total weight of the composition,
   (d) the at least one fatty alcohol is present in an amount in the range of from 2.5 to 20.0 wt%, preferably in the range of from 3.0 to 15.0 wt%, more preferably in the range of from 4.0 to 10.0 wt%, related to the total weight of the composition, and/or
   (e) the at least one non-ionic surfactant is present in an amount in the range of from 0.2 to 5.0 wt%, more preferably in the range of from 0.5 to 4.0 wt%, even more preferably in the range of from 1.0 to 2.5 wt%, related to the total weight of the composition.
86 The cosmetic composition according to any one of embodiments 51 to 85, wherein the composition has a pH of 4.0 or less, preferably in the range of from 1.0 to 3.50, more preferably in the range of from 1.50 to 3.0.
87 The cosmetic composition according to any one of embodiments 51 to 86, wherein the composition has a viscosity at 25°C in the range of from 100 to 2,000 mPa.s, preferably in the range of from 200 to 1,500 mPa.s, more preferably in the range of from 500 to 1,000 mPa.s determined according to the method of the description.
88 The cosmetic composition according to any one of embodiments 51 to 87, wherein the composition comprises the component (d) to the component (e) in a weight ratio in the range of from 10.0:0.5 to 10.0:4.0, more preferably in the range of from 10.0:1.0 to 10.0:3.5, even more preferably in the range of from 10.0:1.5 to 10.0:3.0.
89 The cosmetic composition according to any one of embodiments 51 to 88, wherein the component (d) and the component (e) are comprised in the composition, in part or all, in a gel network system.
90 The cosmetic composition according to any one of embodiments 51 to 89, essentially consisting of, or consisting of:
   (a) glyoxylic acid, pyruvic acid, 2-ketobutyric acid, 2-ketoglutaric acid, glyoxylic acid ester, glyoxylic acid amide, glyoxyloyl acid, glyoxyloyl keratin amino acids, glyoxyloyl carbocysteine, or a mixture thereof,
   (b) stearamidopropyltrimonium methosulfate,
   (c) locust bean gum, tara gum, konjac gum, alginate, tragacanth gum, karaya gum, or a mixture thereof,
   (d) cetyl alcohol, stearyl alcohol, cetearyl alcohol and behenyl alcohol, or a mixture thereof,
   (e) ceteareth-25, steareth-20, PEG hydrogenated castor oil , or a mixture thereof,
   (f) an aqueous carrier consisting essentially of water, or of a water/glycerol mixture,
   (g) optionally, one or more pH adjusters selected from (in)organic Brönsted acids and bases,
   (h) optionally, one or more additives selected from sugar alcohols, preservatives, antioxidants, chelators, electrolytes, pearlescent aids, opacifiers, amino acids/hydrolyzed proteins in a combined amount of 6.0 wt% or less, based on the total weight of the composition,
   wherein the cosmetic compoisition is essentially free of, or free of silicones, colouring agents, perfume oils, fragrances, UV filters, sunscreens.
91 The use of the method the according to any one of embodiments 1 to 50 or of the cosmetic composition according to any one of embodiments 51 to 90, for the semi-permanent deformation of hair.

## Claims

1. A method for the semi-permanent deformation of hair, the method comprising the steps of:
(A) applying a cosmetic composition onto hair,
(B) leaving the composition on the hair for a suitable application time,
(C) rinsing off the composition from the hair, and
(D) applying heat onto the hair using a heating device,
wherein the cosmetic composition comprises:
(a) at least one keto-compound selected from alpha-ketoacids, alpha-ketoacid esters, alpha-ketoacid amides, glyoxyloyl-derivatized compounds, or mixtures thereof,
(b) at least one surfactant selected from mono-tail or double-tail fatty amidoamines or esteramines, quaternary fatty ammonium compounds, quaternary fatty amidoammonium or esterammonium compounds, fatty imidazolines, complex quaternary ammonium compounds, quaternary ammonium compounds of PEG-ylated carbohydrate derivatives,
(c) at least one thickener selected from guar gum, xanthan gum, carrageenan, pectin, agar, locust bean gum, sclerotium gum, tara gum, gellan gum, konjac gum, arabic gum, alginate, tragacanth gum, karaya gum, diutan gum,
(d) at least one fatty alcohol, and
(e) at least one surfactant different from component (b) and (d),
(f) an aqueous carrier.

2. The method according to claim 1, wherein the cosmetic composition essentially consists of, or consists of:
(a) at least one keto-compound selected from alpha-ketoacids, optionally in the form of a salt or hydrate, or alpha-ketoacid esters, or alpha-ketoacid amides, or glyoxyloyl-derivatized compounds, or mixtures thereof,
(b) at least one surfactant selected from mono-tail or double-tail fatty amidoamines or esteramines, quaternary fatty ammonium compounds, quaternary fatty amidoammonium or esterammonium compounds, fatty imidazolines, complex quaternary ammonium compounds, quaternary ammonium compounds of PEG-ylated carbohydrate derivatives,
(c) at least one thickener selected from guar gum, xanthan gum, carrageenan, pectin, agar, locust bean gum, sclerotium gum, tara gum, gellan gum, konjac gum, arabic gum, alginate, tragacanth gum, karaya gum, diutan gum,
(d) at least one fatty alcohol, and
(e) at least one surfactant different from component (b) and (d),
(f) a carrier, wherein the carrier is water or a mixture of water and one or more watersoluble polyhydric alcohols,
(g) optionally, one or more pH adjusters selected from (in)organic Brönsted acids and bases,
(h) optionally, one or more additives in a combined amount of 10.0 wt% or less, based on the total weight of the composition.

3. The method according to claim 1 or 2, wherein the at least one keto-compound (a) is selected from glyoxylic acid, pyruvic acid, glyoxyloyl keratin amino acids, glyoxyloyl carbocysteine, or a mixture thereof.

4. The method according to any one of the preceding claims, wherein the at least one surfactant (b) comprises a mono-tail amidoamine selected from arachidamidopropyldimethylamine, arachidamidopropyldiethylamine, arachidamidoethyldiethylamine, arachidamidoethyldimethylamine, behenamidopropyldiethylamine, behenamidopropyldimethylamine, behenamidoethyldiethylamine, behenamidoethyldimethylamine, brassicamidopropyldimethylamine, lauramidopropyldimethylamine, linoleamidopropyldimethylamine, myristamidopropyldimethylamine, oleamidopropyldimethylamine, palmitamidopropyldimethylamine, stearamidopropyldimethylamine.

5. The method according to any one of the preceding claims, wherein the at least one surfactant (b) comprises a mono-tail or double-tail quaternary amidoammonium compound, wherein the quaternary ammonium cation is selected from babassuamidopropalkonium, behenamidopropyltrimonium, stearamidopropyltrimonium, behenamidopropyl-PPG-dimonium, dilinoleamidopropyldimonium.

6. The method according to any one of the preceding claims, wherein the at least one surfactant (b) comprises a complex quaternary ammonium compound selected from Quaternium-8, Quaternium-33, and Quaternium-91, Polyquat-10, Polyquat-11, Polyquat-22, Polyquat-37.

7. The method according to any one of the preceding claims, wherein the at least one surfactant (b) comprises a quaternary ammonium compound of a PEG-ylated methyl glucose ether, wherein the quaternary ammonium cation is lauryl methyl gluceth-10 hydroxypropyldimonium.

8. The method according to any one of the preceding claims, wherein theat least one thickener (c) is selected from agar, locust bean gum, tara gum, gellan gum, konjac gum, arabic gum, alginate, tragacanth gum, karaya gum.

9. The method according to any one of the preceding claims, wherein the at least one fatty alcohol (d) is selected from the group consisting of linear or branched C14 to C30 fatty alcohols, more preferably from the group consisting of linear C14 to C30 fatty alcohols, even more preferably from the group consisting of cetyl alcohol, stearyl alcohol, cetearyl alcohol and behenyl alcohol.

10. The method according to any one of the preceding claims, wherein the at least one surfactant (e) is a non-ionic surfactant selected from the group consisting of PEG-ylated C8 to C30 alkyl ethers, preferably from the group consisting of PEG-ylated C8 to C30 alkyl ethers having at least 2 ethylene glycol units, or at least 5 ethylene glycol units, more preferably from the group consisting of PEG-ylated C8 to C30 alkyl ethers having from 10 to 50 ethylene glycol units, preferably from the group consisting of PEG-ylated C8 to C30 alkyl ethers having from 15 to 30 ethylene glycol units, more preferably from the group consisting of ceteareth-25 and steareth-20.

11. The method according to any one of the preceding claims, wherein the composition is essentially free of, or free of silicones.

12. The method according to any one of the preceding claims, wherein:
(a) the at least one keto-compound (a) is present in an amount in the range of from 3.0 to 18.0 wt%, preferably in the range of from 5.0 wt% to 17.0 wt%, more preferably in the range of from 10.0 to 16.0 wt%, related to the total weight of the composition,
(b) the at least one quaternary ammonium compound of an PEG-ylated methyl glucose ether is present in an amount in the range of from 0.1 to 10.0 wt%, more preferably in the range of from 0.1 to 5.0 wt%, even more preferably in the range of from 0.5 to 3.0 wt%, related to the total weight of the composition,
(c) the at least one thickener is present in an amount in the range of from 0.01 to 1.0 wt%, preferably in the range of from 0.01 to 0.5 wt%, more preferably in the range of from 0.01 to 0.3 wt%, related to the total weight of the composition,
(d) the at least one fatty alcohol is present in an amount in the range of from 2.5 to 20.0 wt%, preferably in the range of from 3.0 to 15.0 wt%, more preferably in the range of from 4.0 to 10.0 wt%, related to the total weight of the composition, and/or
(e) the at least one non-ionic surfactant is present in an amount in the range of from 0.2 to 5.0 wt%, more preferably in the range of from 0.5 to 4.0 wt%, even more preferably in the range of from 1.0 to 2.5 wt%, related to the total weight of the composition.

13. A cosmetic composition for the semi-permanent deformation of hair, essentially consisting of, or consisting of:
(a) at least one keto-compound selected from alpha-ketoacids, optionally in the form of a salt or hydrate, or alpha-ketoacid esters, or alpha-ketoacid amides, or glyoxyloyl-derivatized compounds, or mixtures thereof,
(b) at least one surfactant selected from mono-tail or double-tail fatty amidoamines or esteramines, quaternary fatty ammonium compounds, quaternary fatty amidoammonium or esterammonium compounds, fatty imidazolines, complex quaternary ammonium compounds, quaternary ammonium compounds of PEG-ylated carbohydrate derivatives,
(c) at least one thickener selected from guar gum, xanthan gum, carrageenan, pectin, agar, locust bean gum, sclerotium gum, tara gum, gellan gum, konjac gum, arabic gum, alginate, tragacanth gum, karaya gum, diutan gum,
(d) at least one fatty alcohol, and
(e) at least one surfactant different from component (b) and (d),
(f) a carrier, wherein the carrier is water or a mixture of water and one or more watersoluble polyhydric alcohols,
(g) optionally, one or more pH adjusters selected from (in)organic Brönsted acids and bases,
(h) optionally, one or more additives in a combined amount of 10.0 wt% or less, based on the total weight of the composition.

14. The cosmetic composition according to claim 13, wherein:
(a) the at least one keto-compound (a) is present in an amount in the range of from 3.0 to 18.0 wt%, preferably in the range of from 5.0 wt% to 17.0 wt%, more preferably in the range of from 10.0 to 16.0 wt%, related to the total weight of the composition,
(b) the at least one quaternary ammonium compound of an PEG-ylated methyl glucose ether is present in an amount in the range of from 0.1 to 10.0 wt%, more preferably in the range of from 0.1 to 5.0 wt%, even more preferably in the range of from 0.5 to 3.0 wt%, related to the total weight of the composition,
(c) the at least one thickener is present in an amount in the range of from 0.01 to 1.0 wt%, preferably in the range of from 0.01 to 0.5 wt%, more preferably in the range of from 0.01 to 0.3 wt%, related to the total weight of the composition,
(d) the at least one fatty alcohol is present in an amount in the range of from 2.5 to 20.0 wt%, preferably in the range of from 3.0 to 15.0 wt%, more preferably in the range of from 4.0 to 10.0 wt%, related to the total weight of the composition, and/or
(e) the at least one non-ionic surfactant is present in an amount in the range of from 0.2 to 5.0 wt%, more preferably in the range of from 0.5 to 4.0 wt%, even more preferably in the range of from 1.0 to 2.5 wt%, related to the total weight of the composition.

15. The cosmetic composition according to claims 13 or 14, essentially consisting of, or consisting of:
(a) glyoxylic acid, pyruvic acid, 2-ketobutyric acid, 2-ketoglutaric acid, glyoxylic acid ester, glyoxylic acid amide, glyoxyloyl acid, glyoxyloyl keratin amino acids, glyoxyloyl carbocysteine, or a mixture thereof,
(b) stearamidopropyltrimonium methosulfate,
(c) locust bean gum, tara gum, konjac gum, alginate, tragacanth gum, karaya gum, or a mixture thereof,
(d) cetyl alcohol, stearyl alcohol, cetearyl alcohol and behenyl alcohol, or a mixture thereof,
(e) ceteareth-25, steareth-20, PEG hydrogenated castor oil , or a mixture thereof,
(f) an aqueous carrier consisting essentially of water, or of a water/glycerol mixture,
(g) optionally, one or more pH adjusters selected from (in)organic Brönsted acids and bases,
(h) optionally, one or more additives selected from sugar alcohols, preservatives, antioxidants, chelators, electrolytes, pearlescent aids, opacifiers, amino acids/hydrolyzed proteins in a combined amount of 6.0 wt% or less, based on the total weight of the composition,
wherein the cosmetic compoisition is essentially free of, or free of silicones, colouring agents, perfume oils, fragrances, UV filters, sunscreens.

16. The use of the method the according to any one of claims 1 to 12 or of the cosmetic composition according to any one of claims 13 to 15, for the semi-permanent deformation of hair.
